# EUROPEAN PATENT APPLICATION

(11) **EP 4 684 806 A1**
(43) Date of publication of application: **28.01.2026**
(21) Application number: 24382796.1
(22) Date of filing: 22.07.2024
(51) Int. Cl.: A61K 47/68, A61P 35/00

(54) **FAP-TARGETED ANTIBODY-DRUG CONJUGATES**

(71) Applicant: Oncomatryx Biopharma, S.L., 48160 Derio (Vizcaya) (ES)
(72) Inventor: EGANA GANUZA, Isabel, Derio, Bizkaia (ES); ALDABA AREVALO, Eneko, Derio, Bizkaia (ES); LON VARA SALAZAR, Yosu, Derio, Bizkaia (ES); LERTXUNDI FERRAN, Josu, Derio, Bizkaia (ES); RICHTER, Wolfgang, Derio, Bizkaia (ES); FABRE, Myriam, Derio, Bizkaia (ES); SIMON BUELA, Laureano, Derio, Bizkaia (ES)
(74) Representative: Mewburn Ellis LLP

(57) **Abstract**

The present invention relates to anti-FAP-targeted antibody-drug conjugates (ADCs), in particular anti-FAP antibody-drug conjugates having the formula A-(L-D)p, or a pharmaceutically acceptable salt or solvate thereof, wherein A is an anti-FAP antibody, L is a linker, D is a drug comprising a topoisomerase inhibitor, and p is 1 to 15. Methods of using such conjugates in the treatment of cancer are also disclosed.

## Description

### Technical Field

The present invention relates to FAP-targeting antibody-drug conjugates (ADCs) comprising a topoisomerase inhibitor as payload, and to their use in medicine, e.g. in the treatment of cancer.

### Background

ADCs represent a promising approach for cancer treatment.¹ They consist of a monoclonal antibody chemically linked to a potent cytotoxic agent through a specialized linker that enables the selective delivery of highly cytotoxic payloads to tumors. This design combines the target specificity and extended circulation time of antibodies with the potent anticancer effects of cytotoxic drugs.² The significant success of ADCs is evidenced by the availability of 11 FDA-approved ADCs for over 20 specific indications and the ongoing evaluation of over 100 ADCs in clinical trials.^{3,4}

The efficacy and safety of an ADC are influenced by each component, requiring careful consideration in the development process. Key factors to be addressed in ADC development include selecting the target antigen, antibody, cytotoxic payload, linker, and conjugation methods.⁵

When it comes to ADC design, there are many options for targets, payloads and linkers, and each component of the ADC must be compatible to achieve high specificity, efficacy and safety (see e.g. Marei et al. Cancer Cell International (2022) 22(1):255). The payloads currently used for ADC development fall into the following major categories: tubulin inhibitors, DNA damaging agents and transcription inhibitors.⁶

The wider used payloads affect microtubules formation. Microtubules represent essential components of the cytoskeleton in eukaryotic cells, serving diverse functions crucial for cell viability and division, including maintaining cell morphology, facilitating organelle transport, and orchestrating cell motility and mitosis. Thereby, they represent prime targets for cancer therapy, given their pivotal roles in tumor cell proliferation.⁷ Tubulin inhibitors, which disrupt microtubule dynamics by binding to tubulin and arresting cells in the G2/M phase of the cell cycle inducing apoptosis in tumor dividing cells, emerged some years ago as potent cytotoxic payloads for ADCs. ⁸ Among them, tubulin polymerization enhancers like auristatin compounds MMAE and MMAF act on the β-subunit of the α,β tubulin dimer, thereby deregulating the growth of microtubules.⁹ In contrast, tubulin polymerization inhibitors, such as tubulysin and analogues, block the polymerization of tubulin dimers by inhibiting the formation of mature microtubules.^{8,10} However, certain tumors, such as some forms of colorectal cancer, exhibit resistance to microtubule inhibitor therapy due to their chromosomal instability.¹¹

Conversely, DNA inhibitors provide a different mode of action in contrast to tubulin inhibitors, as they exert cytotoxic effects throughout the entirety of the cell cycle by causing double-strand DNA breakage, alkylation, chimerism, crosslinking, and other mechanisms. This broader spectrum of action represents another therapeutic alternative for the treatment of solid tumors.¹² For example, different studies have outlined the efficacy of topoisomerase inhibitors such as irinotecan in metastatic colorectal cancer.^{13,14} In this sense, ADCs using deruxtecan as payload have emerged as a promising strategy in cancer therapy.¹⁵

Deruxtecan, a Topoisomerase I inhibitor derived from exatecan, a structural analog of camptothecin, exhibits potent cytotoxic activity against tumor cells by entering the nucleus and causing DNA damage and apoptotic cell death.¹⁶ Clinical trials evaluating trastuzumab deruxtecan-based ADC (Enhertu) have shown promising results in patients with diverse types of cancer, including breast, lung, and gastric cancers, among others. These ADCs have demonstrated notable antitumor activity in HER2+ positive tumors and manageable safety profiles in early-phase clinical trials, leading to their advancement into later-stage trials and regulatory approval for certain indications.¹⁷

However, those ADCs have been engineered to directly target antigens present in tumor cells, restricting their effectiveness to specific groups of patients with antigen-positive tumors.⁵ Additionally, the heterogeneous expression of targets on tumor cells can induce resistance mechanisms.¹⁸ In this sense, clinical resistance to ADCs is intrinsically linked to tumor heterogeneity, which contributes to suboptimal therapeutic responses. Tumor heterogeneity encompasses genetic and phenotypic diversity within a tumor, leading to varying treatment responses and the emergence of ADC-resistant cell populations.¹⁹ To overcome this challenge, recent findings from both preclinical and clinical studies indicate that elements of the tumor stroma may represent promising target antigens for the development of anti-cancer ADC drugs.²⁰

Targeting the tumor stroma, which comprises various supportive elements like fibroblasts, immune cells, and extracellular matrix components, can offer several advantages. Firstly, targeting stroma may overcome the challenges associated with tumor heterogeneity, as stromal elements exhibit greater consistency across different tumor types and stages and is often less genetically diverse and more stable compared to cancer cells, potentially reducing resistance development. ²¹ Secondly, tumor stroma plays pivotal roles in tumor growth, angiogenesis, and immune evasion, making them appropriate targets for therapeutic intervention.²² Among tumor stroma candidates for ADC-targeted therapy, cancer associated fibroblasts (CAFs) emerge as the main candidates due to their overrepresentation in solid tumors.²¹ In this sense, fibroblast activation protein- α (FAP), a type II membrane serine protease with predominant expression in CAFs and minimal expression in healthy tissues, has become a relevant target for ADC therapy.²³ FAP-expressing CAFs are abundant within the tumor microenvironment of solid tumors, and play crucial roles in promoting tumor growth, invasion, and metastasis, as well as immune suppression. Preclinical studies have shown the ability of FAP-targeting ADCs to effectively deliver cytotoxic payloads to FAP-expressing cells, leading to tumor regression in animal models.²⁴⁻²⁶ In clinical trials, FAP-targeting ADCs are being tested for their safety and efficacy in patients with various types of cancer.^{23,26}

The activity of ADCs has been shown to be unpredictable (see, for example, Nguyen et al. Cancers (Basel). (2023) 15(3):713). Each component of the ADC must be compatible to achieve high specificity, efficacy and safety (see *e.g.* Marei et al. Cancer Cell International (2022) 22(1):255).To date, the only effective FAP-targeting ADCs use microtubule inhibitors as payloads, and no FAP-targeting topoisomerase inhibitors have been described.

Here we describe for the first time the development and preclinical efficacy of an ADC that targets FAP with topoisomerase inhibitors as payload.

The present invention has been devised in light of the above considerations.

### Summary of the Invention

Broadly, the present invention relates to anti-FAP antibodies, conjugates thereof and payloads for use in antibody-conjugate strategies. In particular, the present inventors have found that the anti-FAP ADCs described herein exhibit selective binding to the extracellular region of FAP, efficient internalisation, and surprising *in vitro* and *in vivo* efficacy.

Accordingly, in a first aspect, the present disclosure provides A conjugate having the formula I:

A-(L-D)ₚ (I)

or a pharmaceutically acceptable salt or solvent thereof,
wherein:
   A is an antibody comprising
      (i) a heavy chain variable (VH) region comprising the following CDRs:
         HC-CDR1 having the amino acid sequence of SEQ ID NO:7
         HC-CDR2 having the amino acid sequence of SEQ ID NO:8
         HC-CDR3 having the amino acid sequence of SEQ ID NO:9
         or a variant thereof having one amino acid variation in one or more of HC-CDR1, HC-CDR2, or HC-CDR3; and
      (ii) a light chain variable (VL) region comprising the following CDRs:
         LC-CDR1 having the amino acid sequence of SEQ ID NO:10
         LC-CDR2 having the amino acid sequence of SEQ ID NO:11
         LC-CDR3 having the amino acid sequence of SEQ ID NO:12
         or a variant thereof having one amino acid variation in one or more of LC-CDR1, LC-CDR2, or LC-CDR3;
   L is linker
   D is a drug comprising a topoisomerase inhibitor
   p is 1 to 15.

In some embodiments of the first aspect, A comprises:
(i) a heavy chain variable (VH) region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9; and
(ii) a light chain variable (VL) region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12.

In some embodiments of the first aspect, A comprises a heavy chain variable (VH) region comprising an amino acid sequence having at least 90%, 95%, 99% or 100% sequence identity with SEQ ID NO:5. In some embodiments of the first aspect, A comprises a light chain variable (VL) region comprising an amino acid sequence having at least 90%, 95%, 99% or 100% sequence identity with SEQ ID NO:6.

In some embodiments of the first aspect, A comprises a heavy chain comprising an amino acid sequence having at least 90%, 95%, 99% or 100% sequence identity with SEQ ID NO:3. In some embodiments of the first aspect, A comprises a light chain comprising an amino acid sequence having at least 90%, 95%, 99% or 100% sequence identity with SEQ ID NO:4.

In some embodiments of the first aspect, D is of formula II: 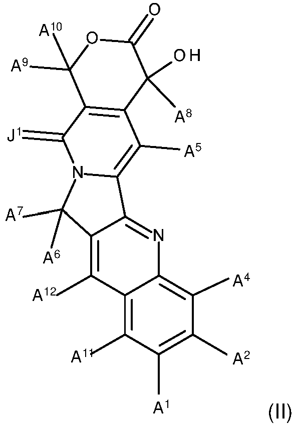
A¹ and A² are each independently selected from the group consisting of halo, hydrogen, C₁-C₃ alkyl, phenyl, hydroxy, C₁-C₃ alkoxy,
or wherein A¹ and A² together with the atoms to which they are bonded form a 5-6 membered fused ring;
either
   A¹¹ is H;
   A¹² is -(C₁₋₆ alkyl)-A³ or A³; or
   A¹¹ and A¹² together with the carbon atoms to which they are attached form a ring substituted with A³ at any position on the ring, preferably the ring structure is 4, 5, 6, 7 or 8 atoms in size, more preferably 5, 6, 7 or 8 atoms in size; preferably the ring is a carbocyclic ring;
A³ is 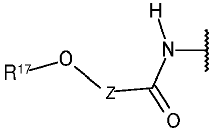
wherein
Z is a bond or a C₁-C₆ alkyl chain or a substituted or unsubstituted aryl group or wherein Z is X₁-Ar, wherein the Ar group is directly attached to the OR¹⁷ group.
wherein X₁ is a C₁-C₆ alkyl chain and Ar is a substituted or unsubstituted aryl group;
A⁴, A⁵, A⁶ A⁷, A⁹ and A¹⁰ are each individually hydrogen or a C₁-C₃ alkyl;
A⁸ is hydrogen or a C₁-C₄ alkyl;
R¹⁷ is a bond, -CH₂X', -CH₂NHX', -CH₂NHC(O)X', -C(O)NHNHC(O)X', -C(O)X', -C(O)NHNHX', -OX', - NHX' or -SX', wherein X' is a bond to linker L and
J¹ is O, S, or Se.

Preferably, Z is a C₁-C₆ alkyl chain. Preferably, Z is a linear C₁-C₆ alkyl chain.

Preferably, wherein A¹ and A² are each independently selected from the group of halo, hydrogen, a linear C₁-C₃ alkyl chain, phenyl, hydroxy, C₁-C₃ alkoxy, or wherein A¹ and A² together with the atoms to which they are bonded form a fused ring.

In some embodiments, D is Exatecan.

In some embodiments of the first aspect, L comprises a protease-cleavable linker, wherein the linker is cleavable by an intracellular protease, optionally wherein the intracellular protease is a cathepsin.

In some embodiments of the first aspect, L comprises a GGFG linker. In some embodiments, L comprises an aminomethoxy group. In some embodiments, L comprises a maleimidocaproyl (MC) group. In some embodiments, L comprises a maleimidocaproyl-GGFG-aminomethoxy linker.

In some embodiments of the first aspect, L comprises a spacer. In some embodiments, the spacer has a chain length of 2 to 30 atoms. In some embodiments, the spacer comprises or consists of an alkylene or oxyalkylene group. In some embodiments, the spacer comprises between one and six ethylene glycol units, e.g. a triethylene glycol.

In some embodiments of the first aspect, L-D has the following structure: 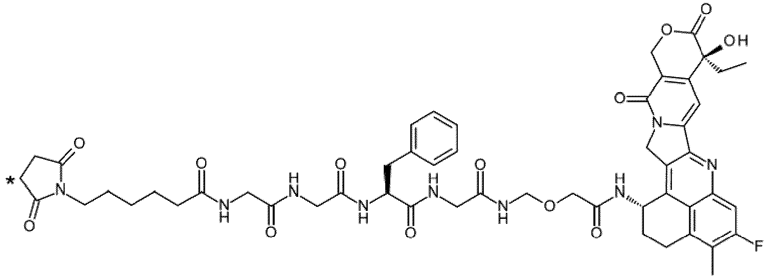 wherein * indicates the site of attachment to A.

In some embodiments of the first aspect, the conjugate is the conjugate described herein as OMTX805.

In some embodiments of the first aspect, p is 5, 6, 7, 8 or 9.

In a second aspect, the present invention provides a pharmaceutical composition comprising a conjugate according to the first aspect, and a pharmaceutically acceptable carrier, excipient or diluent.

In some embodiments of the second aspect, the pharmaceutical composition comprises a plurality of conjugates according to the first aspect. In some embodiments, p is provided as an average of the plurality of conjugates. In some embodiments, when p is provided as an average of the plurality of conjugates, p is between 7 and 9. In some embodiments, p is between 7.5 and 8.5.

In a third aspect, the present invention provides a conjugate according to the first aspect, or a composition according to the second aspect, for use in medicine.

In a fourth aspect, the present invention provides a conjugate according to the first aspect, or a composition according to the second aspect, for use in a method of treatment of cancer in a mammalian subject, or for use in a method of treating an inflammatory or fibrotic condition in a mammalian subject.

In a fifth aspect, the present invention provides a method of treating cancer in a mammalian subject, or a method of treating an inflammatory or fibrotic condition in a mammalian subject, comprising administering a therapeutically effective amount of a conjugate according to the first aspect, or a composition according to the second aspect.

In a sixth aspect, the present invention provides the use of a conjugate according to the first aspect, or a composition according to the second aspect, in the manufacture of a medicament for use in a method of treating cancer in a mammalian subject, or for use in a method of treating an inflammatory or fibrotic condition in a mammalian subject.

In some embodiments of the fourth to the sixth aspects, the cancer comprises a solid tumor. In some embodiments, the cancer is colorectal cancer, sarcoma, fibrosarcoma, leiomyosarcoma, oesophageal cancer, breast cancer, melanoma, gastric cancer, head and neck cancer, ovarian cancer, bladder cancer, lung cancer, pancreatic cancer, cholangiocarcinoma, or mesothelioma. In some embodiments the cancer is colorectal cancer. In some embodiments, the cancer is a colorectal cancer, optionally a colorectal cancer having FAP-expressing tumor cells and/or FAP-expressing stromal cells (e.g. FAP-expressing CAFs). In some embodiments, the cancer is microsatellite-stable (MSS) colorectal cancer.

In some embodiments, the cancer is a metastatic cancer. In some embodiments, the primary cancer of the metastatic cancer comprises colorectal cancer or MSS colorectal cancer. In some embodiments, the metastatic cancer comprises peritoneal metastases or liver metastases. In preferred embodiments, the primary cancer of the metastatic cancer comprises colorectal cancer or MSS colorectal cancer and the metastasis comprises peritoneal metastases or liver metastases.

In some embodiments of the fourth to the sixth aspects, the inflammatory or fibrotic condition is liver fibrosis, lung fibrosis, kidney fibrosis, cardiac fibrosis, Crohn's disease, or arthritis. In some embodiments, the liver fibrosis is metabolic dysfunction-associated steatohepatitis. In some embodiments, the lung fibrosis is idiopathic pulmonary fibrosis. In some embodiments, the arthritis is rheumatoid arthritis.

In some embodiments of the fourth to the sixth aspects, the method comprises simultaneous, sequential, or separate administration of one or more other anti-cancer agents. In some embodiments, the one or more other anti-cancer agents comprise a cytotoxic chemotherapy agent, an anti-angiogenic agent or an immunotherapeutic agent. In some embodiments, the one or more other anti-cancer agents comprise anti-programmed cell death protein 1 (PD-1) antibodies, anti-programmed death-ligand 1 (PD-L1) antibodies or anti-CTLA-4 antibodies. In some embodiments, the one or more other anti-cancer agents comprise Nivolumab (MDX1106) or Pembrolizumab (MK-3475), or Tislelizumab. In some embodiments, the one or more other anti-cancer agents comprises a kinase inhibitor. In some embodiments, the one or more other anti-cancer agents comprises a multi-kinase inhibitor. In some embodiments, the one or more other anti-cancer agents comprise Regorafenib or Pamufetinib (TAS-115).

The invention includes the combination of the aspects and preferred features described except where such a combination is clearly impermissible or expressly avoided.

### Brief Description of the Figures

Embodiments and experiments illustrating the principles of the invention will now be discussed with reference to the accompanying figures in which:
**Figure 1****.** *In vitro* comparative activity of deruxtecan, exatecan, TAM558 and A1B1 through cell viability assay in HT1080-FAP cells. Cell viability (%) over a range of concentrations of each compound.
**Figure 2****.** *In vitro* binding and cytotoxic activity of OMTX805 in HT1080-FAP cells.
**Figure 3A** **and** **B**. **(A)** Tumor growth monitorization in CO-04-272 colorectal tumor patient-derived xenograft mice upon OMTX805 and OMTX705 treatment. Tumor volume (mm³) over time following weekly administration of control Vehicle, OMTX805 (5 and 10 mg/kg) and OMTX705 (5 and 10 mg/kg). **(B)** Tumor volume on day 35 (at the end of the study). Each column represents group mean tumor volume; error bars represent standard error of the mean (SEM). ** *p*<0.01, *** *p*<0.001.
**Figure 4****.** Body weight changes upon treatment with anti-FAP OMTX805 and OMTX705 ADCs in CO-04-272 colorectal tumor patient-derived xenograft mice. Relative murine body weight change over time following administration of control Vehicle, OMTX805 (5 and 10 mg/kg) and OMTX705 (5 and 10 mg/kg).

### Detailed Description

Aspects and embodiments of the present invention will now be discussed with reference to the accompanying figures. Further aspects and embodiments will be apparent to those skilled in the art. All documents mentioned in this text are incorporated herein by reference.

### Definitions

Examples of substituents are described in more detail below.

Unless otherwise stated, halo is selected from chloro (Cl), fluoro (F), bromo (Br) and iodo (I), such as fluoro or chloro.
Hydroxy: -OH.
Phenyl: an aromatic functional group derived from benzene (C₆).

Unless otherwise specified, the term "substituted" as used herein, pertains to a parent group which bears one or more substituents. The term "substituent" is used herein in the conventional sense and refers to a chemical moiety which is covalently attached to, or if appropriate, fused to, a parent group. A wide variety of substituents are well known, and methods for their formation and introduction into a variety of parent groups are also well known. The term "substituted" may refer to a parent group substituted with a C₁₋₆ alkyl, OH, -NH₂, aryl, heterocyclyl, C₁₋₆ alkoxy, -NH-( C₁₋₆ alkyl), halogen.

The term "alkyl" refers to a saturated linear or branched hydrocarbon group, preferably comprising 1 to 20 carbon atoms. The term "alkyl" is used to refer both to monovalent hydrocarbon groups (commonly known as "alkyl" groups) and to divalent hydrocarbon groups (commonly known as "alkylene" groups). The terms "alkyl" and "alkylene" are used interchangeably herein.

The term "C₁₋₆ alkyl" as used herein, pertains to a monovalent or bivalent moiety obtained by removing one or more hydrogen atoms from a carbon atom of a hydrocarbon compound having from 1 to 6 carbon atoms, which are saturated and may also be branched. The term "C₁₋₄ alkyl" as used herein, pertains to a monovalent or bivalent moiety obtained by removing one or more hydrogen atoms from a carbon atom of a hydrocarbon compound having from 1 to 4 carbon atoms, which are saturated.

Examples of saturated alkyl groups include, but are not limited to, methyl (C₁), ethyl (C₂), propyl (C₃), butyl (C₄), pentyl (C₅) and hexyl (C₆).

Alkyl groups may be linear or branched.

Examples of saturated linear alkyl groups include, but are not limited to, methyl (C₁), ethyl (C₂), n-propyl (C₃), n-butyl (C₄), n-pentyl (amyl) (C₅) and n-hexyl (C₆).

Examples of saturated branched alkyl groups include iso-propyl (C₃), iso-butyl (C₄), sec-butyl (C₄), tert-butyl (C₄), iso-pentyl (C₅), and neo-pentyl (C₅).

Alkyl groups may be substituted or unsubstituted. Within the meaning of this invention unless explicitly defined in the formulae the alkyl groups are preferably unsubstituted.
C₁₋₄ alkoxy: The term C₁₋₄ alkoxy as used herein, pertains to an OR group, wherein R is an C₁₋₄ hydrocarbon group. Examples of C₁₋₄ alkoxy groups include, but are not limited to, -OMe, -OEt (ethoxy), -O(nPr) (n-propoxy), -O(iPr) (isopropoxy), -O(nBu) (n-butoxy).
Oxyalkylene: O-alkylene
Fused ring: refers to a C₃₋₁₂ cycloalkyl or C₃₋₁₀ heterocyclyl; preferably a C₃₋₈ cycloalkyl or C₃₋₈ heterocyclyl which is attached to another ring system.

The term "ring" may refer to a carbocyclic ring (e.g. ring consisting of carbon atoms), a heterocyclic ring (e.g. a ring consisting of carbon atoms and heteroatoms). The term "ring" may refer to a 4-8 member ring.

The term "C₃₋₁₂ cycloalkyl" as used herein, pertains to an alkyl group which is also a cyclyl group; that is, a cyclic hydrocarbon (carbocyclic) compound, which moiety has from 3 to 7 carbon atoms, including from 3 to 7 ring atoms. The carbocyclic ring may be saturated or unsaturated.

Examples of cycloalkyl groups include, but are not limited to, those derived from:
saturated monocyclic hydrocarbon compounds:
   cyclopropane (C₃), cyclobutane (C₄), cyclopentane (C₅), cyclohexane (C₆), cycloheptane (C₇), methylcyclopropane (C₄), dimethylcyclopropane (C₅), methylcyclobutane (C₅), dimethylcyclobutane (C₆), methylcyclopentane (C₆), dimethylcyclopentane (C₇) and methylcyclohexane (C₇);
unsaturated monocyclic hydrocarbon compounds:
   cyclopropene (C₃), cyclobutene (C₄), cyclopentene (C₅), cyclohexene (C₆), methylcyclopropene (C₄), dimethylcyclopropene (C₅), methylcyclobutene (C₅), dimethylcyclobutene (C₆), methylcyclopentene (C₆), dimethylcyclopentene (C₇) and methylcyclohexene (C₇); and
saturated polycyclic hydrocarbon compounds:
   norcarane (C₇), norpinane (C₇), norbornane (C₇).

The term "C₃₋₁₀ heterocyclyl" as used herein, pertains to a heterocyclic compound, which has from 3 to 10 ring atoms, of which from 1 to 5 are ring heteroatoms. In certain embodiments, each ring has from 3 to 7 ring atoms, of which from 1 to 4 are ring heteroatoms. The ring may be saturated or unsaturated, and may be bridged or unbridged. The ring may be a fused ring or a single ring. For the avoidance of doubt, substituents on the heterocycloalkyl ring may be linked via either a carbon atom or a heteroatom.

In this context the term 'heteroatom' means O, S, N, Si or B (Boron).

In this context, the prefixes (e.g. C₃₋₁₀ C₃₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

Aryl groups may be a heteroaryl group.

In this context, the prefixes (e.g. C₃₋₁₀ C₃₋₇, C₅₋₆, etc.) denote the number of ring atoms, or range of number of ring atoms, whether carbon atoms or heteroatoms. For example, the term "C₅₋₆ heterocyclyl", as used herein, pertains to a heterocyclyl group having 5 or 6 ring atoms.

Examples of monocyclic heterocyclyl groups include, but are not limited to, those derived from:
N₁: aziridine (C₃), azetidine (C₄), pyrrolidine (tetrahydropyrrole) (C₅), pyrroline (e.g., 3-pyrroline, 2,5-dihydropyrrole) (C₅), 2H-pyrrole or 3H-pyrrole (isopyrrole, isoazole) (C₅), piperidine (C₆), dihydropyridine (C₆), tetrahydropyridine (C₆), azepine (C₇);
O₁: oxirane (C₃), oxetane (C₄), oxolane (tetrahydrofuran) (C₅), oxole (dihydrofuran) (C₅), oxane (tetrahydropyran) (C₆), dihydropyran (C₆), pyran (C₆), oxepin (C₇);
S₁: thiirane (C₃), thietane (C₄), thiolane (tetrahydrothiophene) (C₅), thiane (tetrahydrothiopyran) (C₆), thiepane (C₇);
O₂: dioxolane (C₅), dioxane (C₆), and dioxepane (C₇);
O₃: trioxane (C₆);
N₂: imidazolidine (C₅), pyrazolidine (diazolidine) (C₅), imidazoline (C₅), pyrazoline (dihydropyrazole) (C₅), piperazine (C₆);
N₁O₁: tetrahydrooxazole (C₅), dihydrooxazole (C₅), tetrahydroisoxazole (C₅), dihydroisoxazole (C₅), morpholine (C₆), tetrahydrooxazine (C₆), dihydrooxazine (C₆), oxazine (C₆);
N₁S₁: thiazoline (C₅), thiazolidine (C₅), thiomorpholine (C₆);
N₂O₁: oxadiazine (C₆);
O₁S₁: oxathiole (C₅) and oxathiane (thioxane) (C₆); and,
N₁O₁S₁: oxathiazine (C₆).

The term "aryl," as used herein, represents a monovalent monocyclic, bicyclic, or multicyclic ring system formed by carbon atoms, wherein the ring attached to the pendant group is aromatic. Examples of aryl groups are phenyl, naphthyl, phenanthrenyl, and anthracenyl. An aryl ring can be attached to its pendant group at any heteroatom or carbon ring atom that results in a stable structure and any of the ring atoms can be optionally substituted unless otherwise specified.

Aryl groups may be substituted or unsubstituted. Without wanting to be bound by any theory, within the meaning of this invention, the aryl groups are preferable unsubstituted.

The terms ni-ns are used to refer to the number of repeating structural units, for example
repeating -(OCH₂CH₂)- or -(CH₂)- units.

### Fibroblast activation protein

As used herein "Fibroblast activation protein", "fibroblast activating protein", "FAP" and "FAPα" are used interchangeably. FAP is also known as prolyl endopeptidase FAP. FAP is a member of the dipeptidyl peptidase (DPP) family, which also comprises DPPIV. FAP is a type II transmembrane glycoprotein comprising a short N terminal cytoplasmic region, a transmembrane region, and a large extracellular region which comprises the active site. FAP monomers are not active, but form active homodimers, as well as heterodimers with DPPIV. FAP expression is typically low in normal adult tissues but is highly upregulated in many cancers, including almost all carcinomas (see e.g. Puré and Blomberg, Oncogene (2018) 37(32):4343-4357). FAP expression has also been reported in fibroblast-like synoviocytes (FLSs) in rheumatoid arthritis patients (see, *e.g.,* Bauer et al., Arthritis Res. Therp. (2006):8(6); R171).

The FAP as provided herein includes any forms, homologous or variants of FAP. For example, the FAP may be a FAP of any mammalian species. In some embodiments, FAP is human FAP (also known as Seprase, 170 kDa melanoma membrane-bound gelatinase, fibroblast activation protein alpha or integral membrane serine protease), the amino acid sequence of which is disclosed at UniProt accession No. Q12884 (Version 210, dated 29 May 2024) (SEQ ID NO: 13). In some embodiments, a molecule that binds FAP (e.g. an antibody molecule or a conjugate thereof) may bind to a region of the extracellular domain of FAP. The extracellular domain of human FAP comprises residues 26-760 of the full-length human FAP protein (SEQ ID NO:16). In some embodiments, FAP is murine FAP (also known as fibroblast activation protein alpha or integral membrane serine protease), the amino acid sequence of which is disclosed at UniProt accession No. P97321 (Version 181, dated 29 May 2024) (SEQ ID NO:14). The extracellular domain of murine FAP comprises residues 26-761 of the full-length murine FAP protein (SEQ ID NO:16). The FAP according to the present invention is understood to encompass monomeric, dimeric or multimeric forms of FAP.

### Antibody or antigen-binding fragments thereof

The present invention relates to FAP-targeted antibody-drug conjugates comprising anti-FAP antibodies or antigen-binding fragments thereof.

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb), polyclonal antibodies (including polyclonal antisera), monospecific antibodies and multispecific antibodies (e.g. bispecific antibodies) and antibody fragments (as long as they display binding to the relevant molecule). Antibodies may be human antibodies, humanised antibodies, or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

Antibodies may be intact, or fragments derived from full antibodies. It has been shown that fragments of a whole antibody can perform the function of binding antigens. Thus, reference to antibody herein, and with reference to the methods, arrays and kits of the invention, covers a full antibody and also covers any polypeptide or protein comprising an antibody binding fragment. Examples of binding fragments are (i) an Fab fragment consisting of VL, VH, CL and CH1 domains; (ii) an Fd fragment consisting of the VH and CH1 domains; (iii) a fragment consisting of the VL and CL domains (the light chain) (iv) a Fv fragment consisting of the VL and VH domains of a single antibody; (v) a dAb fragment which consists of a VH domain; (vi) isolated CDR regions; (vii) F(ab')2 fragments, consisting of a bivalent fragment comprising two linked Fab fragments; (viii) single chain Fv molecules (scFv), wherein a VH domain and a VL domain are linked by a peptide linker which allows the two domains to associate to form an antigen binding site; (ix) bispecific single chain Fv dimers (WO 93/11161) and (x) "diabodies" which consist of multivalent or multispecific fragments constructed by gene fusion (WO94/13804; 58). Fv, scFv or diabody molecules may be stabilised by the incorporation of disulphide bridges linking the VH and VL domains. The term "antibody" as used herein also encompasses minibodies, which comprise a scFv joined to a CH3 domain, and nanobodies, which are antibody fragments derived from heavy-chain only IgG antibodies found in the *Camelidae* family.

Antibodies generally comprise six complementarity-determining regions CDRs; three in the heavy chain variable (VH) region: HC-CDR1, HC-CDR2 and HC-CDR3, and three in the light chain variable (VL) region: LC-CDR1, LC-CDR2, and LC-CDR3. The six CDRs together define the paratope of the antibody, which is the part of the antibody that binds to the target antigen.

The VH region and VL region comprise framework regions (FRs) either side of each CDR, which provide a scaffold for the CDRs. From N-terminus to C-terminus, VH regions comprise the following structure: N term-[HC-FR1]-[HC-CDR1]-[HC-FR2]-[HC-CDR2]-[HC-FR3]-[HC-CDR3]-[HC-FR4]-C term; and VL regions comprise the following structure: N term-[LC-FR1]-[LC-CDR1]-[LC-FR2]-[LC-CDR2]-[LC-FR3]-[LC-CDR3]-[LC-FR4]-C term.

The target antigen for an antibody, or antigen-binding fragment thereof, according to the present invention is fibroblast activation protein (FAP) as described herein.

In some embodiments, an antibody described herein comprises, or consists of, a whole antibody which binds to FAP. As used herein, 'whole antibody' refers to an antibody having a structure which is substantially similar to the structure of an immunoglobulin (Ig). Different kinds of immunoglobulins and their structures are described *e.g*. in Schroeder and Cavacini J Allergy Clin Immunol. (2010) 125(202): S41-S52.

Immunoglobulins of type G (*i.e.* IgG) are -150 kDa glycoproteins comprising two heavy chains and two light chains. From N- to C-terminus, the heavy chains comprise a VH followed by a heavy chain constant region comprising three constant domains (CH1, CH2, and CH3), and similarly the light chains comprise a VL followed by a CL. Depending on the heavy chain, immunoglobulins may be classed as IgG *(e.g.* IgG1, IgG2, IgG3, IgG4), IgA (*e.g.* IgA1, IgA2), IgD, IgE, or IgM. The light chain may be kappa (κ) or lambda (λ).

In some embodiments, the antibody comprises, or consists of, an IgG (*e.g.* IgG1, IgG2, IgG3, IgG4), IgA (e.g. IgA1, IgA2), IgD, IgE, or IgM which binds to FAP.

In some embodiments described herein, an amino acid sequence of an antibody or antigen-binding fragment referred to herein (*e.g*. an amino acid sequence of a CDR or VH/VL region) may comprise one or more amino acid variations. That is, an amino acid sequence of an antibody or antigen-binding fragment thereof referred to herein may comprise one or more variant amino acid residues compared to the amino acid residue at the corresponding position of the equivalent, non-variant amino acid sequence. An amino acid variation as described herein may comprise an amino acid deletion, insertion or substitution. That is a variant amino acid sequence having one amino acid variation, as described herein, may comprise one amino acid deletion, insertion, or substitution as compared to the non-variant reference amino acid sequence.

In some embodiments, a variant as described herein comprises a substitution. For example, in some embodiments, an amino acid sequence of an antibody or antigen-binding fragment referred to herein (*e.g*. an amino acid sequence of a CDR or VH/VL) may comprise one or more amino acid substitutions. That is, one or more amino acids of an amino acid sequence referred to herein may be substituted with another amino acid. A substitution comprises substitution of an amino acid residue with a non-identical 'replacement' amino acid residue. A replacement amino acid residue of a substitution according to the present disclosure may be a naturally-occurring amino acid residue (*i.e.* encoded by the genetic code) which is non-identical to the amino acid residue at the relevant position of the equivalent, unsubstituted amino acid sequence. In some embodiments, a replacement amino acid may be a non-naturally occurring amino acid residue. Examples of non-naturally occurring amino acid residues include norleucine, ornithine, norvaline, homoserine, aib, and other amino acid residue analogues such as those described in Ellman, et al., Meth. Enzym. 202 (1991) 301-336.

In some embodiments, a substitution may be biochemically conservative. In some embodiments, where an amino acid to be substituted is provided in one of rows 1 to 5 of the table below, the replacement amino acid of the substitution is another, non-identical amino acid provided in the same row:

| **Row** | **Shared property** | **Amino acids** |
|---|---|---|
| 1 | Hydrophobic | Met, Ala, Val, Leu, Ile, Trp, Tyr, Phe, Norleucine |
| 2 | Neutral hydrophilic | Cys, Ser, Thr, Asn, Gln |
| 3 | Acidic or negatively-charged | Asp, Glu |
| 4 | Basic or positively-charged | His, Lys, Arg |
| 5 | Orientation influencing | Gly, Pro |

In some embodiments, a replacement amino acid in a substitution may have the same side chain polarity as the amino acid residue it replaces. In some embodiments, a replacement amino acid in a substitution may have the same side chain charge (at pH 7.4) as the amino acid residue it replaces (see e.g. Alberts et al. Molecular Biology of the Cell. 4th edition. New York: Garland Science; 2002).

In some embodiments, substitution(s) may be functionally conservative. That is, in some embodiments, the substitution may not affect (or may not substantially affect) one or more functional properties (*e.g*. target binding) of the antigen-binding molecule comprising the substitution as compared to the equivalent unsubstituted molecule.

An antibody or antigen-binding fragment according to the present invention binds FAP. The ability of an antibody or antigen-binding fragment thereof to bind to a given peptide/polypeptide can be analysed by methods well known to the skilled person, including analysis by ELISA, immunoblot (*e.g*. western blot), immunoprecipitation, Surface Plasmon Resonance (SPR; see *e.g*. Hearty et al., Methods Mol Biol (2012) 907:411-442) or Bio-Layer Interferometry (see *e.g*. Lad et al., (2015) J Biomol Screen 20(4): 498-507).

An antibody according to the present invention may bind to human FAP (*i.e.* SEQ ID NO:13). An antibody according to the present invention may bind to murine FAP (*i.e.* SEQ ID NO:14). In some embodiments, an antibody according to the present disclosure displays cross-species reactivity. That is, an antibody according to the present disclosure may bind to FAP from multiple different species. In some embodiments, an antibody according to the present invention binds to human FAP (*i.e.* SEQ ID NO:13) and to murine FAP (*i.e.* SEQ ID NO:14).

In some embodiments, an antibody according to the present invention binds the extracellular region of FAP. For example, an antibody according to the present invention may bind to human FAP in the region corresponding to positions 26 to 760 of SEQ ID NO:13 (*i.e.* SEQ ID NO:15); for example, an antibody according to the present invention may bind to murine FAP in the region corresponding to positions 26 to 761 of SEQ ID NO:14 (*i.e.* SEQ ID NO:16).

An antibody according to the present invention may bind to the extracellular region of human FAP (*i.e*. SEQ ID NO:15) and to the extracellular region of murine FAP (*i.e*. SEQ ID NO:16).

The region of a peptide/polypeptide to which an antibody binds can be determined by the skilled person using various methods well known in the art, including X-ray co-crystallography analysis of antibody-antigen complexes, peptide scanning, mutagenesis mapping, hydrogen-deuterium exchange analysis by mass spectrometry, phage display, competition ELISA and proteolysis-based 'protection' methods. Such methods are described, for example, in Gershoni et al., BioDrugs, 2007, 21(3):145-156.

In some embodiments, an antibody or antigen-binding fragment thereof according to the present invention comprises:
a VH region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9,
or a variant thereof comprising one or two or three amino acid variations in one or more of HC-CDR1, HC-CDR2, or HC-CDR3; and
a VL region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12;
or a variant thereof comprising one or two or three amino acid variations in one or more of LC-CDR1, LC-CDR2 or LC-CDR3.

In some embodiments, an antibody or antigen-binding fragment thereof according to the present invention comprises:
a VH region comprising the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9,
or a variant thereof comprising one amino acid variation in one or more of HC-CDR1, HC-CDR2,
or HC-CDR3; and
a VL region comprising the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12;
or a variant thereof comprising one amino acid variation in one or more of LC-CDR1, LC-CDR2 or LC-CDR3.

In some embodiments, an antibody or antigen-binding fragment thereof according to the present invention comprises:
a VH region incorporating the following CDRs:
   HC-CDR1 having the amino acid sequence of SEQ ID NO:7
   HC-CDR2 having the amino acid sequence of SEQ ID NO:8
   HC-CDR3 having the amino acid sequence of SEQ ID NO:9; and
a VL region incorporating the following CDRs:
   LC-CDR1 having the amino acid sequence of SEQ ID NO:10
   LC-CDR2 having the amino acid sequence of SEQ ID NO:11
   LC-CDR3 having the amino acid sequence of SEQ ID NO:12.

In some embodiments, the antibody or antigen-binding fragment thereof comprises:
a VH region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:5.

In some embodiments, the antibody or antigen-binding fragment thereof comprises:
a VL region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:6.

In some embodiments, the antibody or antigen-binding fragment thereof comprises:
a VH region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:5 and a VL region comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity to the amino acid sequence of SEQ ID NO:6.

In some embodiments, the antibody or antigen-binding fragment thereof comprises a VH region comprising an amino acid sequence having at least 90%, 95% or 99% sequence identity to the amino acid sequence of SEQ ID NO:5.

In some embodiments, the antibody or antigen-binding fragment thereof comprises a VL region comprising an amino acid sequence having at least 90%, 95% or 99% sequence identity to the amino acid sequence of SEQ ID NO:6.

In some embodiments, the antibody or antigen-binding fragment thereof comprises a VH region comprising an amino acid sequence having at least 90%, 95% or 99% sequence identity to the amino acid sequence of SEQ ID NO:5 and a VL region comprising an amino acid sequence having at least 90%, 95% or 99% sequence identity to the amino acid sequence of SEQ ID NO:6.

In some embodiments, the antibody or antigen-binding fragment thereof comprises a VH region comprising the amino acid sequence of SEQ ID NO:5.

In some embodiments, the antibody or antigen-binding fragment thereof comprises a VL region comprising the amino acid sequence of SEQ ID NO:6.

In some embodiments, the antibody or antigen-binding fragment thereof comprises a VH region comprising the amino acid sequence of SEQ ID NO:5 and a VL region comprising the amino acid sequence of SEQ ID NO:6.

In some embodiments, the antibody or antigen-binding fragment thereof comprises a heavy chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:3.

In some embodiments, the antibody or antigen-binding fragment thereof comprises a light chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:4.

In some embodiments, the antibody or antigen-binding fragment thereof comprises a heavy chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:3 and a light chain comprising an amino acid sequence having at least 70% sequence identity, more preferably one of at least 75%, 80%, 85%, 86%, 87%, 88%, 89%, 90%, 91%, 92%, 93%, 94%, 95%, 96%, 97%, 98%, 99%, or 100%, sequence identity with SEQ ID NO:4.

In some embodiments, the antibody or antigen-binding fragment thereof comprises a heavy chain comprising an amino acid sequence having at least 90%, 95% or 99% sequence identity with SEQ ID NO:3.

In some embodiments, the antibody or antigen-binding fragment thereof comprises a light chain comprising an amino acid sequence having at least 90%, 95% or 99% sequence identity with SEQ ID NO:4.

In some embodiments, the antibody or antigen-binding fragment thereof comprises a heavy chain comprising an amino acid sequence having at least 90%, 95% or 99% sequence identity with SEQ ID NO:3 and a light chain comprising an amino acid sequence having at least 90%, 95% or 99% sequence identity with SEQ ID NO:4.

In some embodiments, the antibody or antigen-binding fragment thereof comprises a heavy chain comprising the amino acid sequence of SEQ ID NO:3.

In some embodiments, the antibody or antigen-binding fragment thereof comprises a light chain comprising the amino acid sequence of SEQ ID NO:4.

In some embodiments, the antibody or antigen-binding fragment thereof comprises a heavy chain comprising the amino acid sequence of SEQ ID NO:3 and a light chain comprising the amino acid sequence of SEQ ID NO:4.

In some embodiments, the antibody or antigen-binding fragment thereof comprises, or consists of:
one or more (e.g. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:3.

In some embodiments, the antibody or antigen-binding fragment thereof comprises, or consists of:
one or more (*e.g*. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:4.

In some embodiments, the antibody or antigen-binding fragment thereof comprises, or consists of:
(i) one or more (*e.g*. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:3; and
(ii) one or more (*e.g*. two) polypeptides comprising, or consisting of, an amino acid sequence having at least 70%, preferably one of 75%, 80%, 85%, 90%, 91%, 92%, 93%, 94%, 95%, 96%, 97%, 98%, 99% or 100% amino acid sequence identity to the amino acid sequence of SEQ ID NO:4.

In some embodiments, the antibody or antigen-binding fragment thereof comprises, or consists of one or more (*e.g*. two) polypeptides comprising, or consisting of, an amino acid sequence having the amino acid sequence of SEQ ID NO:3.

In some embodiments, the antibody or antigen-binding fragment thereof comprises, or consists of one or more (*e.g*. two) polypeptides comprising, or consisting of, an amino acid sequence having the amino acid sequence of SEQ ID NO:4.

In some embodiments, the antibody or antigen-binding fragment thereof comprises, or consists of:
(i) one or more (*e.g*. two) polypeptides comprising, or consisting of, an amino acid sequence having the amino acid sequence of SEQ ID NO:3; and
(ii) one or more (*e.g*. two) polypeptides comprising, or consisting of, an amino acid sequence having the amino acid sequence of SEQ ID NO:4.

The antibody or antigen-binding fragment thereof according to the invention may be characterised by reference to certain functional properties.

In some embodiments, the antibody or antigen-binding fragment thereof according to the present invention exhibits selective binding to human and mouse FAP. For example, the antibody or antigen-binding fragment thereof according to the present invention demonstrates binding to human and mouse FAP with EC50 values of < 5 nM, < 4 nM, < 3 nM, < 2 nM or < 1 nM. In some embodiments, the antibody or antigen-binding fragment thereof according to the present invention exhibits binding to human and mouse FAP with EC50 values of < 1 nM, < 0.5 nM, < 0.4 nM, or < 0.3 nM.

The binding of an antibody for its target antigen can be determined by the skilled person using various methods well known in the art, including ELISAs, for example as described in Example 2 of the present application.

In some embodiments, the antibody or antigen-binding fragment thereof according to the present invention is internalised into a target cell expressing FAP, following binding of the antibody or antigen-binding fragment thereof to FAP on the target cell's surface. In some embodiments, internalisation of the antibody or antigen-binding fragment thereof according to the present invention, when measured in a suitable internalisation assay, exhibits internalisation into ≥ 50%, ≥60%, ≥ 70%, ≥ 80%, ≥ 85% of target cells within ≤ 120 minutes, ≤ 90 minutes, ≤ 60 minutes of administration of the antibody or antigen-binding fragment thereof. In some embodiments, internalisation of the antibody or antigen-binding fragment thereof into ≥ 85% of target cells occurs in ≤ 60 minutes of administration of the antibody or antigen-binding fragment thereof.

The internalization of an antibody or antigen-binding fragment thereof into target cells can be determined by the skilled person using various methods well known in the art, for example, indirect immunofluorescence (see, *e.g.* Riedl et al. J Biomol Screen. (2016) 21(1):12-23).

The antibody or antigen-binding fragment thereof according to the present invention and their constituent polypeptides may additionally comprise a signal sequence (also known as a leader sequence or signal peptide). Signal sequences normally consist of a sequence of 5-30 hydrophobic amino acids, which form a single alpha helix. Secreted proteins and proteins expressed at the cell surface often comprise signal sequence. Signal sequences are known for many proteins, and are recorded in databases such as GenBank, UniProt and Ensembl, and/or can be identified/predicted e.g. using amino acid sequence analysis tools such as SignalP (Petersen et al., 2011 Nature Methods 8: 785-786) or Signal-BLAST (Frank and Sippl, 2008 Bioinformatics 24: 2172-2176).

The signal sequence may be present at the N-terminus of the polypeptide and may be present in the newly synthesised polypeptide. The signal sequence provides for efficient trafficking of the polypeptide. Signal sequences are often removed by cleavage, and thus are not comprised in the mature polypeptide.

Signal sequences are known for many proteins, and are recorded in databases such as GenBank, UniProt, Swiss-Prot, TrEMBL, Protein Information Resource, Protein Data Bank, Ensembl, and InterPro, and/or can be identified/predicted *e.g*. using amino acid sequence analysis tools such as SignalP (Petersen et al., 2011 Nature Methods 8: 785-786) or Signal-BLAST (Frank and Sippl, 2008 Bioinformatics 24: 2172-2176).

The antibody or antigen-binding fragment thereof according to the present disclosure may comprise the signal sequence shown in SEQ ID NO:17.

### Topoisomerase inhibitors

The present invention relates to FAP-targeted antibody-drug conjugates comprising a topoisomerase inhibitor.

As used herein, the term "topoisomerase inhibitor" refers to the group of compounds that block the action of topoisomerases. Topoisomerases are a group of enzymes that catalyse changes in the topological state of DNA, and play essential roles in DNA replication, transcription, chromosome segregation and recombination. Topoisomerases can be divided into two subtypes: type I topoisomerases and type II topoisomerases. Type I isomerases catalyse reactions involving transient single-stranded breaks. Type II isomerases catalyse reactions involving transient double-strand breaks. The structure and activity of topoisomerases is reviewed, for example, in Sutormin et al. Acta Naturae. (2021) 13(1):59-75 and Baker et al. Nucleic Acids Res. (2009) 37(3):693-701.

The ability of an agent to inhibit topoisomerase activity can be determined by the skilled person using various methods well known in the art, for example, as described in Nitiss et al. Curr Protoc Pharmacol. (2021) 1(10):e250 doi: 10.1002/cpz1.250.

Type I topoisomerases function by attacking the phosphodiester bond in the backbone of DNA to form a transient topoisomerase-DNA intermediate. This allows for rotation of the cleaved DNA strand around the helical access before the topoisomerase re-ligates the cleaved strand to reform duplex DNA. Topoisomerase type I inhibitors generally inhibit topoisomerase I activity by stabilising the topoisomerase-DNA intermediate, thereby preventing DNA re-ligation. The mechanism of type I topoisomerase inhibitors is reviewed, for example, in Pommier et al. Chem Biol. (2020). 17(5):421-433.

In some embodiments, the topoisomerase inhibitor according to the present invention is a type I topoisomerase inhibitor. Type I topoisomerase inhibitors include: camptothecin and structural analogues thereof, and non-camptothecins.

Non-camptothecin type I topoisomerase inhibitors include: indenoisoquinolines, for example, indotecan (LMP400, drugbank accession number (DB) 18752), indimitecan (LMP776) and LMP744 (MJ-!!!65, NSC 706744); phenanthridine derivatives, for example, Topovale (ARC-111), nitidine, fagaronine (see *e.g*. Wang et al. Chem Res Toxicol. (1993) 6(6):813-818); indolocarbazoles, for example, NB-506, edotecarin (DB04882), BMS-250749. In some embodiments, the topoisomerase inhibitor according to the present invention is a non-camptothecin type I topoisomerase inhibitor.

In some embodiments, the topoisomerase inhibitor according to the present invention is a camptothecin (DB04690) or structural analogue thereof. As used herein, the term 'structural analogue' (also referred to as 'chemical analogue', 'analogue' or 'derivative') refers to compounds having a structure similar to that of another compound, but different from it in respect to a certain component (*e.g*. in one or more atoms or functional groups).

Camptothecin analogues include: homocamptothecins (such as Diflomotecan (DB16138)), Gimatecan (DB06721), Topotecan (DB01030), Irinotecan (DB00762), Rubitecan (DB06159), 9-Amino-20(S)-camptothecin (9-AC), Lurtotecan (DB12222), Prothecan (PEG-camptothecin), Cositecan (DB05806), Silatecan (DB12384), Exatecan (DB12185).

In some embodiments, the topoisomerase inhibitor according to the present invention is of formula IIa: 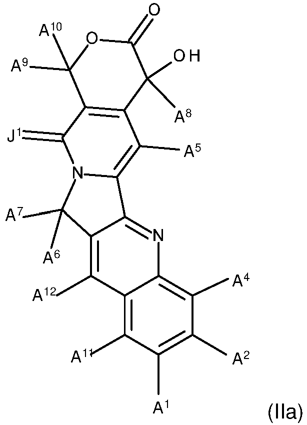
A¹ and A² are each independently selected from the group consisting of halo, hydrogen, C₁-C₃ alkyl, phenyl, hydroxy, C₁-C₃ alkoxy,
or wherein A¹ and A² together with the atoms to which they are bonded form a 5-6 membered fused ring;
either
   A¹¹ is H;
   A¹² is -(C₁₋₆ alkyl)-A³ or A³; or
   A¹¹ and A¹² together with the carbon atoms to which they are attached form a ring substituted with A³ at any position on the ring, preferably the ring structure is 4, 5, 6, 7 or 8 atoms in size, more preferably 5, 6, 7 or 8 atoms in size; preferably the ring is a carbocyclic ring;
A³ is 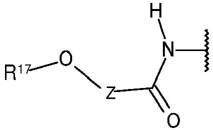
wherein
Z is a bond or a C₁-C₆ alkyl chain or a substituted or unsubstituted aryl group or wherein Z is X₁-Ar, wherein the Ar group is directly attached to the OR¹⁷ group.
wherein X₁ is a C₁-C₆ alkyl chain and Ar is a substituted or unsubstituted aryl group;
A⁴, A⁵, A⁶ A⁷, A⁹ and A¹⁰ are each individually hydrogen or a C₁-C₃ alkyl;
A⁸ is hydrogen or a C₁-C₄ alkyl;
R¹⁷ is a bond, -CH₂X', -CH₂NHX', -CH₂NHC(O)X', -C(O)NHNHC(O)X', -C(O)X', -C(O)NHNHX', -OX', - NHX' or -SX', wherein X' is a bond to linker L and
J¹ is O, S, or Se.

Preferably, Z is a C₁-C₆ alkyl chain. Preferably, Z is a linear C₁-C₆ alkyl chain.

Preferably, wherein A¹ and A² are each independently selected from the group of halo, hydrogen, a linear C₁-C₃ alkyl chain, phenyl, hydroxy, C₁-C₃ alkoxy, or wherein A¹ and A² together with the atoms to which they are bonded form a fused ring.

In some embodiments, the topoisomerase inhibitor is of formula IIIa. 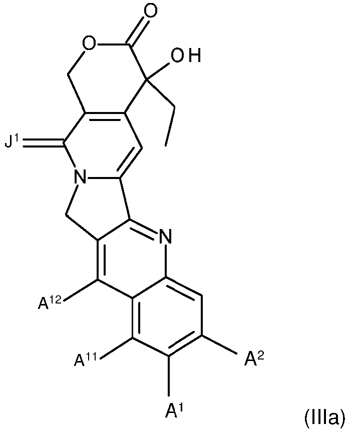 wherein:
A¹ is a C₁-C₃ alkyl chain or H;
A² is halo or H;
or A¹ and A² together with the carbon atoms to which they are attached form a 5-6 member heterocyclic ring;
J¹ is O, S, or Se
either
A¹¹ is H
A¹² is -(C₁₋₃ alkyl)-A³ or A³;
or A¹¹ and A¹² together with the carbon atoms to which they are attached form a 5-8 membered carbocyclic ring substituted with A³;
A³ is 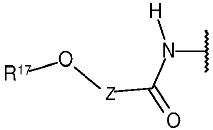
wherein
Z is a bond or a C₁-C₃ alkyl chain and R¹⁷ is a bond, -CH₂X', -CH₂NHC(O)X', -CH₂NHX', - C(O)NHNHC(O)X', -C(O)X', -C(O)NHNHX', -OX', -NHX' or -SX', wherein X' is a bond to linker L.

Preferably, R¹⁷ is -CH₂X', -CH₂NHX', -C(O)NHNHC(O)X', -C(O)X', -C(O)NHNHX', -OX', -NHX' or -SX', wherein X' is a bond to linker L.

Preferably, A¹ is a linear C₁-C₃ alkyl chain. Preferably, Z is a linear C₁-C₃ alkyl chain.

In some embodiments, the topoisomerase inhibitor according to the present invention is TC1, or a pharmaceutically acceptable salt, hydrate, solvate, or crystalline form thereof. In some embodiments, the topoisomerase inhibitor according to the present invention has the following structure: 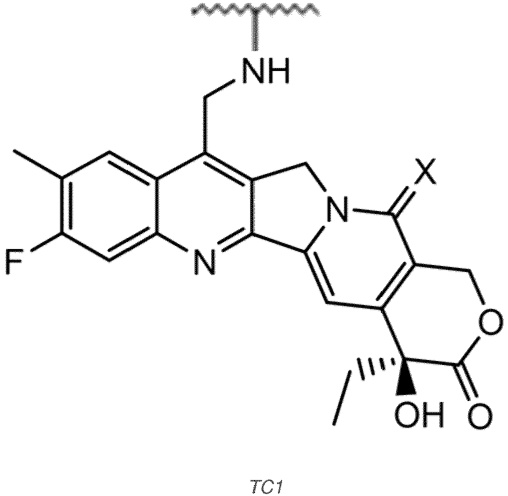
wherein X is O, S, Se;
wherein 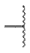 denotes the point of attachment to the linker or attachment to the group 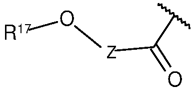
wherein
Z is a bond or a C₁-C₃ alkyl chain and R¹⁷ is a bond, -CH₂X', -CH₂NHC(O)X', -CH₂NHX', - C(O)NHNHC(O)X', -C(O)X', -C(O)NHNHX', -OX', -NHX' or -SX', wherein X' is a bond to linker L.

In some embodiments, the topoisomerase inhibitor according to the present invention is TC2, or a pharmaceutically acceptable salt, hydrate, solvate, or crystalline form thereof. In some embodiments, the topoisomerase inhibitor according to the present invention has the following structure: 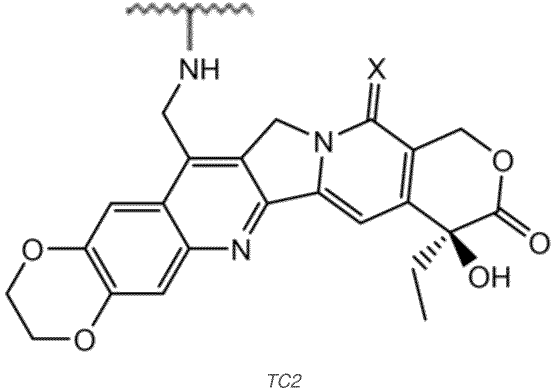
wherein X is O, S, Se;
wherein 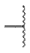 denotes the point of attachment to the linker or attachment to the group 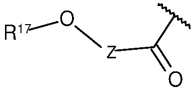
wherein
Z is a bond or a C₁-C₃ alkyl chain and R¹⁷ is a bond, -CH₂X', -CH₂NHX', -CH₂NHC(O)X', - C(O)NHNHC(O)X', -C(O)X', -C(O)NHNHX', -OX', -NHX' or -SX', wherein X' is a bond to linker L..

In some embodiments, the topoisomerase inhibitor according to the present invention is of formula IIb: 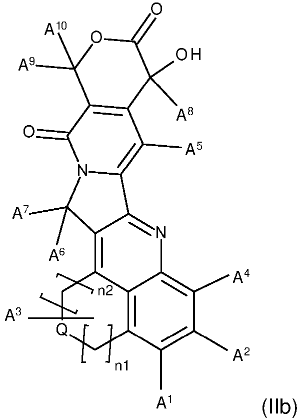 wherein:
A¹ and
A² are each independently selected from the group consisting of halo, hydrogen, C₁-C₃ alkyl, phenyl, hydroxy, C₁-C₃ alkoxy,
or wherein A¹ and A² together with the atoms to which they are bonded form a 5-6 membered fused ring; Q is O, S or CR^{a}R^{b}; wherein R^{a} and R^{b} are each independently selected from hydrogen or a C₁-C₃ alkyl;
n₁ and n₂ are each individually 0, 1 or 2 and wherein n₁ + n₂ is at least 1;
A³ is 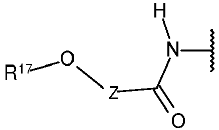
wherein
Z is a bond or a C₁-C₆ alkyl chain or a substituted or unsubstituted aryl group or wherein Z is X₁-Ar, wherein the Ar group is directly attached to the OR¹⁷ group,
wherein X₁ is a C₁-C₆ alkyl chain and Ar is a substituted or unsubstituted aryl group;
A⁴, A⁵, A⁶ A⁷, A⁹ and A¹⁰ are each individually hydrogen or a C₁-C₃ alkyl;
A⁸ is hydrogen or a C₁-C₄ alkyl;
R¹⁷ is a bond, -CH₂X', -CH₂NHX', -C(O)NHNHC(O)X', -C(O)X', -CH₂ NH C(O)X', -C(O)NHNHX', -OX', - NHX' or -SX', wherein X' is a bond to linker L;
wherein A³ represents a group which may be attached at any point on the ring structure containing Q.

Preferably, Z is a C₁-C₆ alkyl chain. Preferably, Z is a linear C₁-C₆ alkyl chain. Preferably, wherein Q is O, S or CR^{a}R^{b} and wherein R^{a} and R^{b} are each independently selected from hydrogen or a linear C₁-C₃ alkyl chain.

Preferably, wherein A¹ and A² are each independently selected from the group of halo, hydrogen, a linear C₁-C₃ alkyl chain, phenyl, hydroxy, C₁-C₃ alkoxy, or wherein A¹ and A² together with the atoms to which they are bonded form a fused ring.

The ring structure containing substituent Q may be 4, 5, 6, 7 or 8 atoms in size depending on the values of n₁ and n₂. Preferably, the ring containing substituent Q is 5, 6, 7 or 8 atoms in size.

In some cases, in accordance with the first aspect of the present invention, the topoisomerase inhibitor is of formula IIIb: 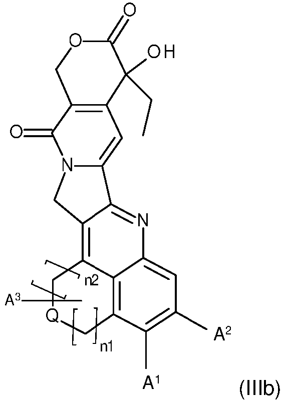 wherein:
A¹ is a C₁-C₃ alkyl chain;
A² is halo;
Q is O, S or CH₂
n₁ and n₂ are each individually 0, 1 or 2 and wherein n₁ + n₂ is at least 1;
A³ is 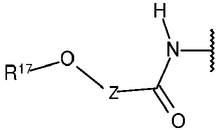
wherein
Z is a bond or a C₁-C₃ alkyl chain and R¹⁷ is a bond, -CH₂X', -CH₂NHX', -CH₂NHC(O)X', - C(O)NHNHC(O)X', -C(O)X', -C(O)NHNHX', -OX', -NHX' or -SX', wherein X' is a bond to linker L.

Preferably, R¹⁷ is -CH₂X', -CH₂NHX', -C(O)NHNHC(O)X', -C(O)X', -C(O)NHNHX', -OX', -NHX' or -SX', wherein X' is a bond to linker L.

Preferably, A¹ is a linear C₁-C₃ alkyl chain. Preferably, Z is a linear C₁-C₃ alkyl chain.

In certain cases, the topoisomerase inhibitor is preferably (1S,9S)-1-Amino-9-ethyl-5-fluoro-9-hydroxy-4-methyl-1,2,3,9,12,15-hexahydro-10H,13H-benzo[de]pyrano[3',4':6,7]indolizino[1,2-b]quinoline-10,13-dione, which corresponds to a compound according to formula IV: 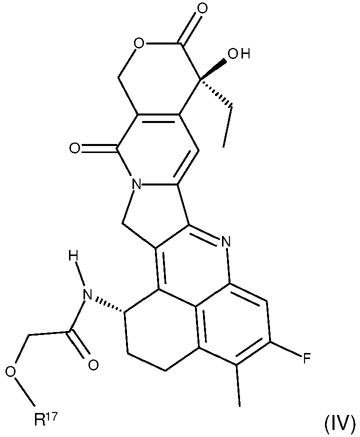 wherein:
R¹⁷ is a bond, -CH₂X', -CH₂NHX', -CH₂NHC(O)X', -C(O)NHNHC(O)X', -C(O)X', -C(O)NHNHX', -OX', - NHX' or -SX', wherein X' is a bond to linker L. Preferably, R¹⁷ is CH₂X', -CH₂NHX', -C(O)NHNHC(O)X', - C(O)X', -C(O)NHNHX', -OX', -NHX' or -SX', wherein X' is a bond to linker L.

In some embodiments, the topoisomerase inhibitor according to the present invention is Exatecan or a derivative thereof. In some embodiments, the topoisomerase inhibitor is Exatecan, or a pharmaceutically acceptable salt, hydrate, solvate, or crystalline form thereof. In preferred embodiments, the topoisomerase inhibitor according to the present invention has the following structure: 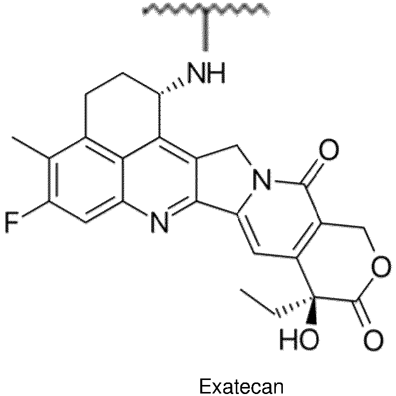
Wherein 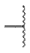 denotes the point of attachment to the linker or attachment to the group 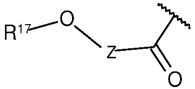
wherein
Z is a C₁-C₃ alkyl chain and R¹⁷ is a bond, -CH₂X', -CH₂NHX', -C(O)NHNHC(O)X', -C(O)X', -C(O)NHNHX', -OX', -NHX' or -SX', wherein X' is a bond to linker L.

### Linker

The term "linker" as used herein refers to the component linking the antibody and the drug. That is, the linker according to the present invention refers to the component of the conjugate of the present invention that connects the anti-FAP antibody and the topoisomerase inhibitor.

In some embodiments, the linker according to the present invention is a cleavable linker. Cleavable linkers are cleaved to release the drug payload *(i.e.* the topoisomerase inhibitor) to the target location *(i.e.* the target cell, tissue *etc.*)*.*

In some embodiments, the linker according to the present invention is an enzymatically cleavable linker. In some embodiments, the linker is a peptide-based linker. In some embodiments, the linker is a protease-cleavable linker, optionally a lysosomal protease-cleavable linker. In some embodiments, the linker is cleaved by an intracellular protease. In some embodiments, the linker is cleaved by a cathepsin.

In some embodiments, the linker according to the present invention comprises a valine-citrulline unit. Valine-citrulline (Val-Cit) is a protease sensitive dipeptide that is cleaved intracellularly by cathepsins (*i.e.* cathepsin B).

In some embodiments, the linker according to the present invention comprises a tetrapeptide. In some embodiments, the linker comprises a glycine-glycine-phenylalanine-glycine tetrapeptide (*i.e.* a GGFG tetrapeptide).

In some embodiments, the linker according to the present invention comprises a self-immolative unit. That is, in some embodiments, the linker comprises a group that undergoes self-immolative elimination to facilitate release of the drug payload (*i.e.* the topoisomerase inhibitor) to the target location (*i.e.* the target cell, tissue *etc.*)*.* In some embodiments, the linker comprises an aminomethoxy group. In some embodiments, the self-immolative unit comprises a para-substituted aminobenzyl group. In some embodiments, the self-immolative unit comprises a para-aminobenzyl ether group. In some embodiments, the linker comprises a para-aminobenzylcarbamate group. In some embodiments, the linker comprises a para-aminobenzylcarbonate group.

In some embodiments, the linker is coupled to the antibody via cysteine-based conjugation. In some embodiments, the linker comprises a thiol-reactive group. In some embodiments, the linker is coupled to the antibody via reaction between cysteine residues in the antibody and a thiol-reactive functional group in the linker. In some embodiments the linker comprises a maleimide group. In some embodiments, the linker comprises a maleimidocaproyl group. The maleimidocaproyl group according to the present invention is reacted with a thiol group of a cysteine residue of the antibody according to the present invention to form a sulphur-carbon bond, thereby effecting linkage of the linker to the antibody. In some embodiments, L comprises a maleimidocaproyl-GGFG-aminomethoxy linker. In some embodiments, L comprises a maleimidocaproyl-GGFG-aminomethyl linker. In some embodiments, L comprises a maleimidocaproyl-valine-citrulline-p-aminobenzylcarbamate linker.

In some embodiments, the linker is selected from the group consisting of: 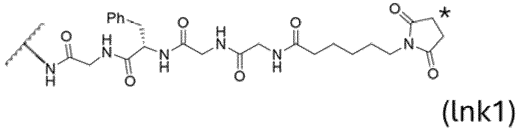 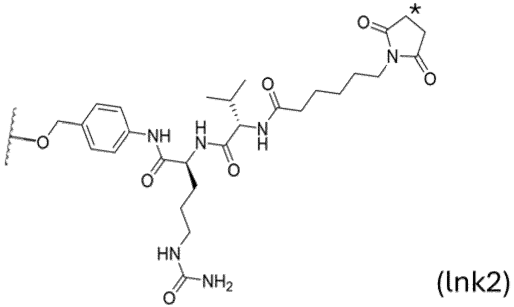 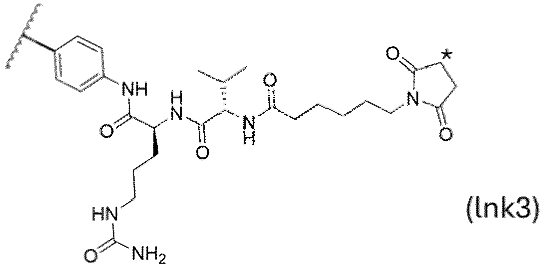 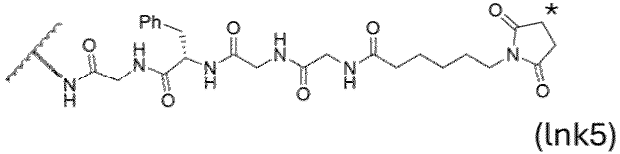 Wherein 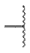 denotes the point of attachment to the R¹⁷, wherein * indicates the site of attachment to A In some embodiments, the linker according to the present invention further comprises a spacer. Spacers are sometimes required due to the bulky nature of payload moieties. Commonly employed spacer moieties include hemiaminal groups, PEG groups, polar acyl sulfamide groups, polar carbamoyl sulfamide groups and HydraSpace (described *e.g.* in Verkade et al., Antibodies (Basel) (2018) 7(1):12 and WO 2016/053107 A1).

In some embodiments, the spacer has a chain length of 2 to 30 atoms. In some embodiments, the spacer comprises or consists of an alkylene (*i.e.* divalent alkyl) or heteroalkylene (*i.e.* divalent heteroalkyl) group. In some embodiments, the spacer comprises or consists of an alkylene or oxyalkylene group. In some embodiments, the spacer comprises or consists of a group (CH₂)ₙ or (OCH₂CH₂)ₙ, wherein n ≥ 1. In some embodiments, the spacer comprises or consists of a group (OCH₂CH₂)ₙ, wherein n ≥ 1. In particular, n may be 1 to 15, 1 to 10, 1 to 6, or 2 to 5. For example, n may be 3 or 4. In some embodiments, the spacer comprises between one and six ethylene glycol units, e.g. a triethylene glycol.

In some cases, R¹⁷ is a bond, -CH₂X', -CH₂NHX', -CH₂NHC(O)X', -C(O)NHNHC(O)X', -C(O)X', - C(O)NHNHX', -OX', -NHX' or -SX', wherein X' is a bond to linker L. In some cases, R¹⁷ is -C(O)X', - C(O)NHNHX', -OX', -NHX' or -SX', wherein X' is a bond to linker L.

In some cases, the linker L further comprises a spacer. In some cases, the spacer has a chain length of 2 to 30 atoms. In some cases, the spacer comprises or consists of an oxyalkylene or alkylene group. In some cases, the spacer comprises or consists of a group -(OCH₂CH₂)ₙ- or -(CH₂)ₙ-, wherein n ≥ 1. In some cases, n = 1 to 15, 1 to 10, 2 to 9, 1 to 6, 2 to 5 or 3 to 5. In certain cases, n = 3 or 4. In certain cases, n = 1, n = 2, n = 3, n = 4, n = 5, n = 6, n = 7, n = 8, n=9orn= 10.

In some cases, the spacer is attached to group R¹⁷ via a bridging group (i.e. the spacer further comprises a bridging group) or is directly attached to the group R¹⁷. In some cases, the spacer further comprises the group-C(O)X' bridging group, wherein X' is a bond to R¹⁷. In some cases, R¹⁷ is CONHNHX' and the spacer is attached to group R¹⁷ via a -C(O)X' bridging group, wherein X' represents the bond between the spacer and R¹⁷. Preferably, R¹⁷ is CONHNHX' and the spacer is a -(OCH₂CH₂)ₙ- attached to R¹⁷ via a -C(O)X' bridging group, wherein n = 2, 3 or 4. In some places in this application R¹⁷ is depicted as being both part of the drug structure and part of the linker. Without wanting to be bound by any theory the chemical structures according to the present invention do not contain groups corresponding to R¹⁷- R¹⁷. For example, when R¹⁷ is CONHNHX' this moiety is only present once at the joining point between the linker and the drug structure. In some cases, the spacer is attached to the linker, L, via a second bridging group. In some cases, the second bridging group is C(O)X" or NHC(O)X", where X" is the bond to the linker, L. In some cases, the linker second bridging group moiety is:

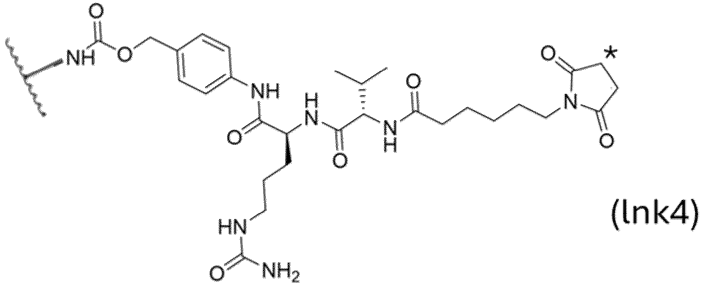

Wherein 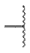 denotes the point of attachment to the R¹⁷, wherein * indicates the site of attachment to A.

In some embodiments, the conjugate according to the present invention comprises Exatecan and a linker, wherein the linker comprises an aminomethoxy group, a GGFG tetrapeptide and a maleimidocaproyl group. In some embodiments the conjugate according to the present invention comprises Deruxtecan or a pharmaceutically acceptable salt, hydrate, solvate, or crystalline form thereof.

In some embodiments, the conjugate according to the present invention comprises the following structure: 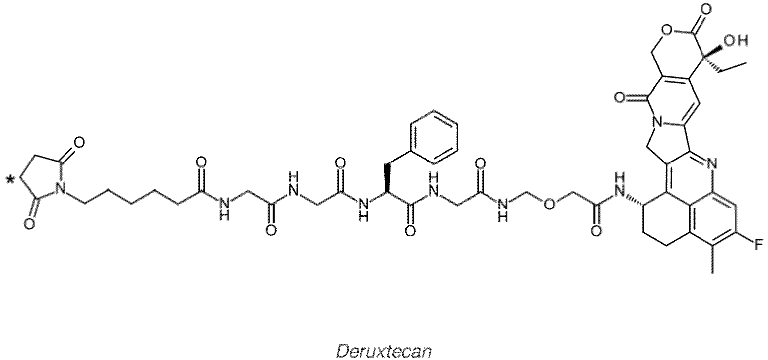 wherein * indicates the site of attachment to A (the antibody).

In some embodiments, the conjugate according to the present invention comprises a structure selected from the following: 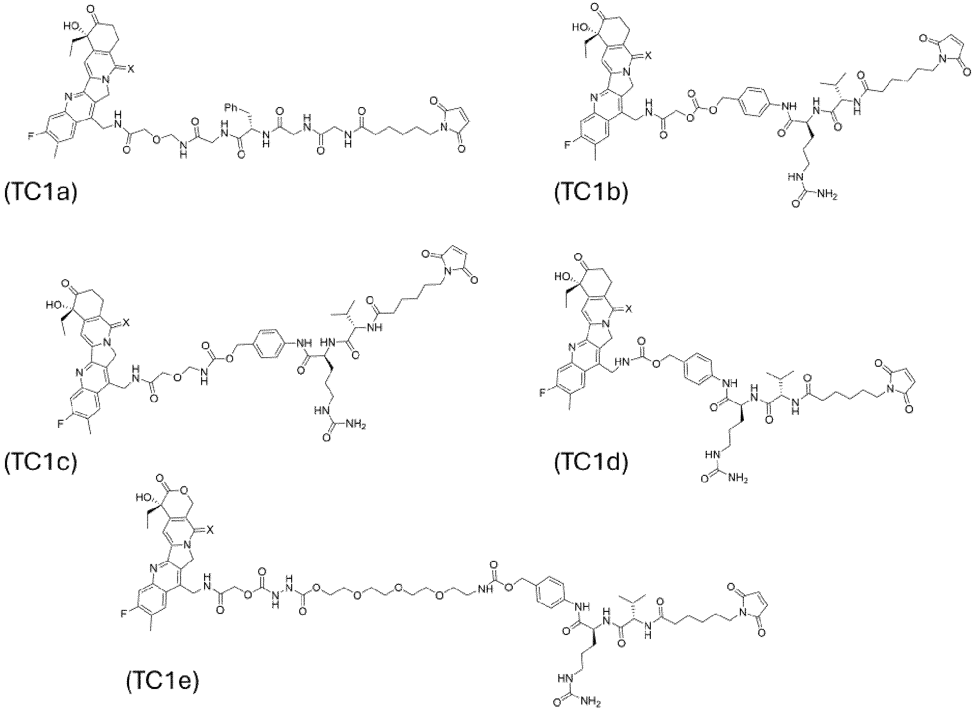 wherein X in the structures TC1a to TC1e is O, S, Se.

In some embodiments, the conjugate according to the present invention comprises a structure selected from the following: 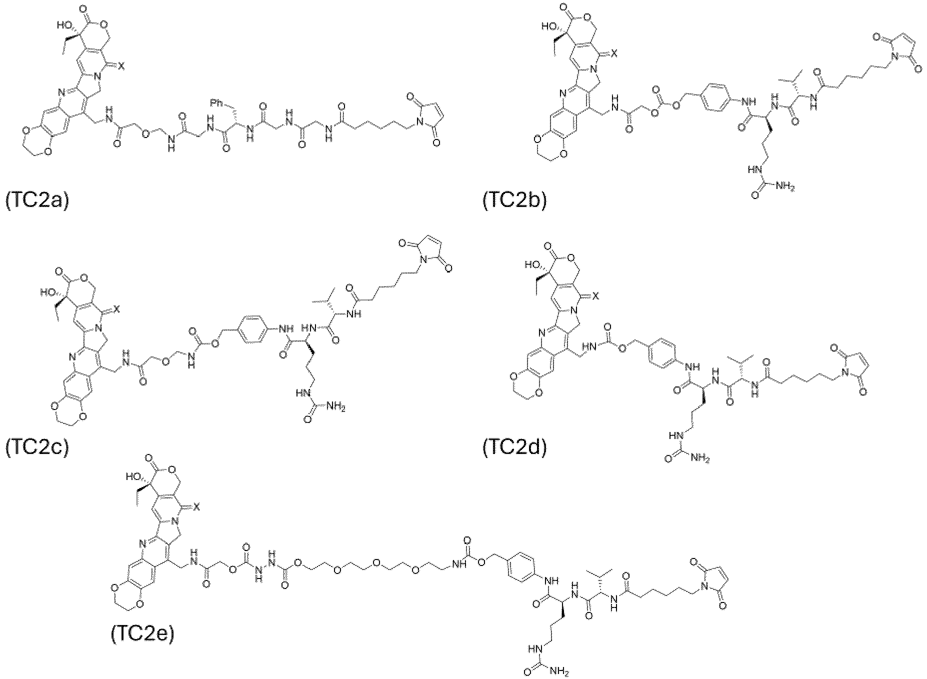 wherein X in the structures TC2a to TC2e is O, S, Se.

In some embodiments, the conjugate according to the present invention comprises a structure selected from the following: 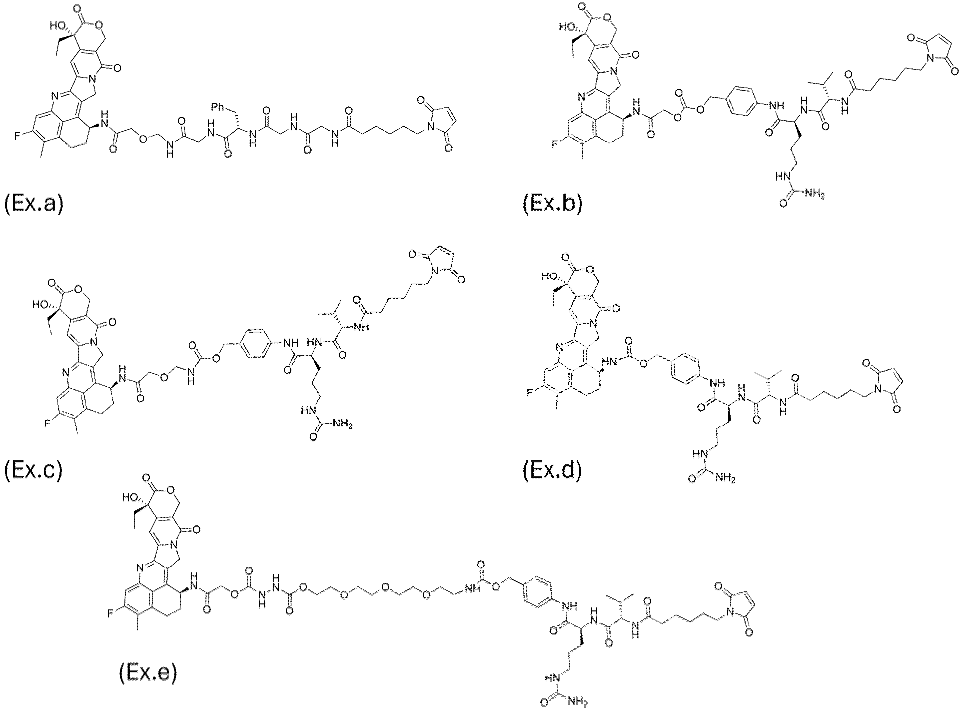 wherein the terminal maleamide group is modified to attach to the antibody, for example by attachment of A (the antibody) at one of the double bond carbons and reduction of the other double bond carbon.

### Anti-cancer agents

The term "anti-cancer agents" as provided herein refers to biologically active agents which are useful in the treatment of cancer.

In some embodiments, in accordance with any aspect of the present invention, the conjugate of the invention may be administered with, or may be for administration with, (whether simultaneously, sequentially or separately) other anti-cancer drugs, including, but not limited to, a cytotoxic chemotherapeutic agent or an anti-angiogenic agent or an immunotherapeutic agent.

Cytotoxic chemotherapeutic agents are well known in the art and include agents such as:
Alkylating agents including nitrogen mustards (for example, bendamustine, chlorambucil, cyclophosphamide, ifosfamide, mechlorethamine/chlormethine, melphalan, uramustine); ethylenimines and methylmelamine derivatives (for example altretamine, thiotepa); expoxides (for example dianhydrogalacititol, dibromodulcitol), alkyl sulfonates (for example, busulfan, hepsulfan), nitrosoureas (for example, carmustine, lomustine, streptozocin, semustine), triazenes (for example dacarbazine, procarbazine, temozolomide), quinone-containing/bioreductive alkylating agents (for example, mitomycin C). Platinum-based chemotherapeutic agents or platinum-based anti-neoplastic agents (also known as "alkylating-like" agents) including Cisplatin, Carboplatin, Dicycloplatin, Eptaplatin, Lobaplatin, Miriplatin, Nedaplatin, Oxaliplatin, Picoplatin, Satraplatin, Triplatin tetranitrate. Antimetabolites including folic acid analogues such as methotrexate (amethopterin); pyrimidine analogues such as fluorouracil (5-fluorouracil; 5-FU), floxuridine (fluorodeoxyuridine; FudR) and cytarabine (cytosine arabinoside); and purine analogues and related inhibitors such as mercaptopurine (6-mercaptopurine; 6-MP), thioguanine (6-thioguanine; TG) and pentostatin (2'-deoxycofonnycin). Natural Products including vinca alkaloids such as vinblastine (VLB), vincristine and vinorelbine; antibiotics such as anthracenediones (e.g. mitoxantrone and anthracycline), dactinomycin (actinomycin D), daunorabicin (daunomycin; rubidomycin), doxorubicin, bleomycin, plicamycin (mithramycin) and mitomycin (mitomycin Q; enzymes such as L-asparaginase; and biological response modifiers such as interferon alphenomes. Monoclonal antibodies including Alemtuzumab, Atezolizumab, Avelumab, Belantamab, Bevacizumab, Blinatumomab, Brentuximab, Cemiplimab, Cetuximab, Daratumumab, Dinutuximab, Dostarlimab, Durvalumab, Elotuzumab, Enfortumab, Gemtuzumab, Inotuzumab Ozogamicin, Ipilimumab, Mogamulizumab, Moxetumomab Pasudotox, Necitumumab, Nivolumab, Ofatumumab, Olaratumab, Panitumumab, Pembrolizumab, Pertuzumab, Polatuzumab Vedotin, Ramucirumab, Rituximab, Sacituzumab Govitecan, Teclistamab, Tisotumab Vedotin, Tositumomab, Trastuzumab, Trastuzumab Deruxtecam, Trastuzumab Emtansine, Tremelimumab. Protein kinase inhibitors including Abemaciclib, Acalabrutinib, Adagrasib, Afatinib, Alectinib, Alpelisib, Asciminib, Axitinib, Binimetinib, Bortezomib, Bosutinib, Brigatinib, Cabozantinib, Carfilzomib, Ceritinib, Cobimetinib, Copanlisib, Crizotinib, Dabrafenib, Dacomitinib, Dasatinib, Duvelisib, Enasidenib, Encorafenib, Entrectinib, Erdafitinib, Erlotinib, Fedratinib, Futibatinib, Gefitinib, Gilteritinib, Glasdegib, Ibrutinib, Idelalisib, Imatinib, Infigratinib, Ivosidenib, Ixazomib, Lapatinib, Larotrectinib, Lenvatinib, Lorlatinib, Midostaurin, Mobocertinib, Neratinib, Nilotinib, Niraparib, Olaparib, Olutasidenib, Osimertinib, Palbociclib, Pamufetinib (TAS-115), Pazopanib, Pemigatinib, Pexidartinib, Ponatinib, Regorafenib, Ribocicib, Ripretinib, Rucaparib, Ruxolitinib, Selumetinib, Sonidegib, Sorafenib, Sunitinib, Talazoparib, Trametinib, Trilaciblib, Umbralisib, Vandetanib, Vemurafenib, Vismodegib, Zanubrutinib. Taxanes including paclitaxel (also known as Taxol) and docetaxel (also known as Taxotere), cabazitaxel (also known as Jevtana). Topoisomerase inhibitors including etoposide, irinotecan, teniposide, topotecan. Hormone agonists/antagonists including antiandrogens (*e.g*. Abiraterone, Apalutamide, Bicalutamide, Cyproterone, Enzalutamide, Flutamide, Nilutamide flutamide and tamoxifen); antiestrogens (*e.g*. aromatase inhibitors, Anastrozole, Exemestane, Fulvestrant, Letrozole, Raloxifene, Tamoxifen, Toremifene); gonadotropin releasing hormone analogues (*e.g*. Degarelix, Goserelin, Histrelin, Leuprolide, Relugolix, Triptorelin); and peptide hormones (*e.g*. Lanreotide, Octreotide, Pasireotide). Miscellaneous agents including substituted urea such as hydroxyurea; and adrenocortical suppressant such as mitotane (o, p'-DDD) and aminoglutethimide.

Anti-angiogenic agents are well known in the art and include anti-cancer agents such as bevacizumab, itraconazole, and carboxyamidotriazole.

Immunotherapeutic agents are known in the art and include, for example, anti-programmed cell death protein 1 (PD-1) antibodies and anti-programmed death-ligand 1 (PD-L1) antibodies, including Nivolumab (MDX1106) and Pembrolizumab (MK-3475) and Tislelizumab.

In some embodiments, in accordance with any aspect of the present invention, the one or more other anti-cancer drugs, comprise a kinase inhibitor. In some embodiments, the kinase inhibitor is a multi-kinase inhibitor. That is, in some embodiments, the kinase inhibitor may have specificity for multiple (*i.e*. two or more) kinases. In some embodiments, the multi-specific kinase inhibitor is Regorafenib or Pamufetinib (TAS-115).

### Subject

The mammalian subject in accordance with aspects of the present disclosure may be any mammal or human. A subject may therefore be a rat, mouse, feline, canine, equine, porcine, ovine, bovine, primate or human. The subject is preferably human. The subject may be male or female. The subject may be a patient. A subject may have been diagnosed with a disease/condition described herein requiring treatment, may be suspected of having such a disease/condition, or may be at risk of developing/contracting such a disease/condition.

In some embodiments, the subject may be a human diagnosed with or classified as being at risk of developing a cancer. In some embodiments, the subject is a human patient having cancer. In certain embodiments, the subject may be a laboratory animal, *e.g*., a mouse model of a cancer.

In certain embodiments, the subject may be a mammal (*e.g*. a human) that has been diagnosed with or classified as being at risk of developing an inflammatory condition, as described herein.

### Treatment

Conjugates according to the present invention and compositions comprising such agents may be provided for use in methods of medical treatment. Treatment may be provided to subjects having a disease or condition in need of treatment. The disease or condition may be a cancer. The disease or condition may be an inflammatory or fibrotic disease or condition.

In some embodiments, the cancer may comprise cells/tissue/stroma/tumor microenvironment which expresses FAP (*i.e.* the cancer may be a FAP-positive cancer). For example, the cancer may be treated by targeted delivery of the cytotoxic payload of the present invention (*i.e.* the topoisomerase inhibitor) by specific recognition of FAP within cells/tissue/stroma/tumor microenvironment of the cancer by the anti-FAP antibody of the invention. In some embodiments, the antibody or antigen-binding fragment thereof according to the present invention binding to FAP may facilitate receptor-mediated internalisation of the conjugate of the present invention, which may then be cleaved by intracellular proteases to release the topoisomerase inhibitor, thereby inducing cytotoxicity.

In some embodiments, the cancer is a cancer expressing FAP (*i.e.* a FAP-positive cancer). In some embodiments, the cancer is a cancer with upregulated FAP expression (*i.e.* in comparison with a comparable non-cancerous cell/tissue/organ/organ system). In some embodiments, a "cancer expressing FAP" includes any cancer comprising cells/tissue/stroma/tumor microenvironment encoding or expressing FAP. In some embodiments, a "cancer with upregulated FAP expression" includes any cancer comprising cells/tissue/stroma/tumor microenvironment expressing FAP at an upregulated level, *e.g*. as compared to the level of expression by comparable non-cancerous cells/tissue/stroma/tumor microenvironment.

In some embodiments, the cancer is a FAP-positive colorectal cancer, that is, in some embodiments, the cancer is a colorectal cancer having FAP-expressing tumor cells and/or FAP-expressing stromal cells (*e.g*. FAP-expressing CAFs).

The treatment may be aimed at prevention of the development or progression of a cancer. As such, the conjugates may be used to formulate pharmaceutical compositions or medicaments and subjects may be prophylactically treated against development of a disease state. This may take place before the onset of symptoms of the disease state, and/or may be given to subjects considered to be at greater risk of development or progression of cancer.

It will be clear to the person skilled in the art that the therapeutic utility of the present invention extends to essentially any disease/condition which would benefit from targeted killing of FAP-expressing cells. The therapeutic utility of the present invention extends to any subject suffering from a condition comprising cells with upregulated FAP expression (*i.e.* as compared to equivalent cells in a healthy subject).

A cancer may be determined to express FAP by any suitable means, which are well known to the skilled person, *e.g*. based on analysis of a biological sample. A cancer expressing FAP may be identified by detection of expression of FAP. Expression may be gene expression or protein expression. Gene expression can be determined *e.g*. by detection of mRNA encoding FAP, for example by quantitative real-time PCR (qRT-PCR). Protein expression can be determined *e.g*. by detection of FAP, for example by antibody-based methods, for example by western blot, immunohistochemistry, immunocytochemistry, flow cytometry, ELISA. Herein, "a cancer expressing FAP" includes any cancer comprising cells/tissue/stroma/tumor microenvironment encoding or expressing FAP. In some embodiments, the cells/tissue/stroma may be a cells/tissue/stroma of a tumor.

In some embodiments, the conjugates according to the present invention and compositions comprising such agents may be provided for use in a method of treatment of cancer in a mammalian subject. In some embodiments, a cancer may be any cancer. In some embodiments a cancer may comprise a solid tumor, for example, a colorectal tumor, a fibrosarcoma, a gastric tumor, a glioblastoma, a renal tumor, a hepatic tumor, a pulmonary tumor, a melanoma, a nasopharyngeal tumor, an oral tumor, an osteosarcoma, an ovarian tumor, a pancreatic tumor, a brain tumor, or a prostatic tumor. Alternatively, the cancer may comprise a blood cancer or haematological cancer, such as a lymphoma, a leukaemia, or a myeloma.

In particular, the cancer may be a colorectal cancer, sarcoma (for example, fibrosarcoma), leiomyosarcoma, oesophageal cancer, breast cancer, melanoma, gastric cancer, head and neck cancer, ovarian cancer, bladder cancer, cholangiocarcinoma, or mesothelioma. In preferred embodiments, the cancer is a colorectal cancer. In preferred embodiments, the cancer is a colorectal cancer having FAP-expressing tumor cells and/or FAP-expressing stromal cells (*e.g*. FAP-expressing CAFs).

In some embodiments, the cancer is microsatellite-stable (MSS) colorectal cancer.

In some embodiments, the cancer is not pancreatic cancer. In some embodiments, the cancer is not lung cancer. In some embodiments, the cancer is not non-small cell lung cancer (NSCLC). In some embodiments, the cancer is not pancreatic cancer or NSCLC.

In some embodiments, the cancer is a metastatic cancer. In some embodiments, the primary cancer of the metastatic cancer comprises colorectal cancer. In some embodiments, the primary cancer of the metastatic cancer comprises MSS colorectal cancer. In some embodiments, the metastasis comprises peritoneal metastases or liver metastases,
In the context of the present invention, the term primary cancer refers to the anatomical site where the cancer first starts growing in the subject. That is, reference to, for example, a primary cancer comprising colorectal cancer means that the cancer first started growing in the subject in the colon or rectum. In such embodiments, the primary site of the cancer is the colon or rectum.

As used herein, the term "metastatic cancer" refers to a primary cancer where the cancer has spread from the primary cancer to another anatomical site in the subject. Thus, in the context of the present invention, metastatic cancer comprises the primary cancer and cancer in an anatomical site of the subject different to the site of the primary cancer. These cancers in sites different to the site of the primary cancer may otherwise be referred to as metastases or metastasis. For the avoidance of doubt, it will be appreciated that reference to "metastases" refers to a plurality of metastasis, *i.e.* a plurality of secondary tumours which are located in one or more anatomical sites of the subject different to the site of the primary cancer.

Where a cancer is referred to as, for example, "metastatic colorectal cancer", this will be understood to refer to a cancer where the primary cancer is colorectal cancer, but this has spread to anatomical sites other than the colon/rectum in the subject. In such examples, the subject comprises both the primary cancer and metastases in anatomical locations different to that of the primary cancer.

In some embodiments, the anti-FAP conjugates described herein may be for useful in the treatment of an inflammatory disease. FAP has been associated with fibrosis, in particular fibrotic conditions involved the liver (*e.g.* cirrhosis), lung (*e.g.* idiopathic pulmonary fibrosis) and colon (*e.g.* Crohn's disease). FAP has also been associated with arthritis, in particular rheumatoid arthritis.

In some embodiments, conjugates according to the present invention, and compositions comprising such agents, are for use in a method of treating an inflammatory disease. In some embodiments, the inflammatory disease is fibrosis, or a disease/condition characterised by fibrosis (*i.e.* a disease/condition in which fibrosis is a symptom). In some embodiments, the inflammatory disease, fibrosis, or disease/condition characterised by fibrosis, may be of the liver, lung, kidney, cardiovascular system (*e.g*. the heart), and/or gastrointestinal system (*e.g*. the colon).

In some embodiments, the disease/condition is of the liver. Such diseases/conditions include: chronic liver disease, liver fibrosis, metabolic dysfunction-associated steatotic liver disease, metabolic dysfunction-associated steatohepatitis, cirrhosis, steatohepatitis, alcoholic liver disease, alcoholic fatty liver, alcoholic hepatitis, alcoholic steatohepatitis, primary biliary cirrhosis, schistosomal liver disease. In some embodiments, the disease/condition is metabolic dysfunction-associated steatotic liver disease (MASLD), previously known as non-alcoholic fatty liver disease. In some embodiments, the disease/condition is metabolic dysfunction-associated steatohepatitis (MASH).

In some embodiments, the disease/condition is of the respiratory system (*e.g*. the lungs). Such diseases/conditions include: pulmonary fibrosis, interstitial lung disease, idiopathic interstitial pneumonia, idiopathic pulmonary fibrosis, cystic fibrosis, scleroderma, asbestosis, chronic obstructive pulmonary disease, chronic bronchitis, asthma, emphysema. In some embodiments, the disease/condition is pulmonary fibrosis. In some embodiments, the inflammatory disease is idiopathic pulmonary fibrosis (IPF).

In some embodiments, the disease/condition is of the cardiovascular system. Such diseases/conditions include: cardiac or myocardial fibrosis, atherosclerosis, cardiomyopathy, peripheral vascular disease, fibrotic vascular disease, myocarditis, interstitial fibrosis, subepicardial fibrosis.

In some embodiments, the disease/conditoin is of the kidney. Such diseases/conditions include: nephritis, glomerulonephritis, tubulointerstitial renal fibrosis, glomerular fibrosis, glomerulosclerosis, diabetic nephropathy, chronic kidney disease.

In some embodiments, the disease/condition is of the gastrointestinal system (*e.g*. the colon). Such diseases/conditions include: inflammatory bowel disease, intestinal fibrosis, ulcerative colitis and Crohn's disease.

In some embodiments, the inflammatory disease is arthritis, in particular rheumatoid arthritis.

### Dosages and method of administration

Multiple doses of the conjugate may be provided. One or more, or each, of the doses may be accompanied by simultaneous, sequential or separate administration of another anti-cancer agent.

In some embodiments the time interval between administration steps performed sequentially is one of at least 12 hours, 24 hours, 36 hours, 48 hours, 72 hours, 4 days, 5 days, 7 days, 10 days, 14 days, 18 days, 21 days, or 28 days, or 1, 2, 3, 4, 5, or 6 months.

In embodiments where administration steps comprise plural, separate introductions of material into a subject, time intervals provided herein may be between the final introduction of one administration step, and the first introduction of the subsequent administration step.

In some embodiments, administration steps of the methods of the present disclosure may be performed simultaneously. That is, in some embodiments an administration step as described herein is performed at the same time as or immediately before/after another administration step as described herein. The time interval between administration steps performed simultaneously is preferably less than one of 72 hours, 48 hours, 36 hours, 24 hours, 12 hours, or 6 hours.

Preferably, the conjugate of the present invention, or a pharmaceutical composition comprising the conjugate according to the present invention is administered via injection. Such administration may be both via infusion (continuous) or bolus (discrete) administration. The method of administration via injection may be, for example, subcutaneous, intradermal, intramuscular, intravenous, intraarterial, intracardiac, intrathecal, intraspinal, intracapsular, subcapsular, intra-orbital, intraperitoneal, intratracheal, subcuticular, intraarticular, subarachnoid, and intra-sternal injection. Preferably, the administration is by intravenous infusion or intravenous injection (bolus administration). More preferably, the administration is by intravenous infusion.

Administration of the articles according to the present disclosure is preferably in a 'therapeutically-effective' or 'prophylactically-effective' amount, this being sufficient to show therapeutic/prophylactic benefit to the subject. The actual amount administered, and rate and time-course of administration, will depend on the nature and severity of the disease/condition and the particular article administered. Prescription of treatment, *e.g.* decisions on dosage *etc.,* is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

### Pharmaceutical compositions

The conjugates of the present invention may be provided in the form of a composition comprising the conjugate. Such compositions may be pharmaceutical compositions or medicaments suitable for clinical use, and may additionally comprise pharmaceutically-acceptable carriers, diluents, excipients or adjuvants.

A pharmaceutical composition according to the present invention may comprise, in addition to the conjugate as described herein, one or more other pharmaceutically acceptable ingredients well known to those skilled in the art, including, but not limited to: pharmaceutically acceptable carriers, diluents, excipients, adjuvants, buffers, pH modifiers, preservatives, anti-oxidants, bacteriostats, stabilisers, suspending agents, solubilisers, surfactants (*e.g*., wetting agents), colouring agents, and isotonicising solutes (*i.e.*, which render the formulation isotonic with the blood, or other relevant bodily fluid, of the intended recipient).

The term 'pharmaceutically-acceptable' as used herein pertains to compounds, ingredients, materials, compositions, dosage forms, etc., which are, within the scope of sound medical judgment, suitable for use in contact with the tissues of the subject in question (*e.g*. a human subject) without excessive toxicity, irritation, allergic response, or other problem or complication, commensurate with a reasonable benefit/risk ratio. Each carrier, diluent, excipient, adjuvant, filler, buffer, preservative, anti-oxidant, lubricant, binder, stabiliser, solubiliser, surfactant, masking agent, colouring agent, flavouring agent or sweetening agent of a composition according to the present disclosure must also be 'acceptable' in the sense of being compatible with the other ingredients of the formulation. Suitable carriers, diluents, excipients, adjuvants, fillers, buffers, preservatives, anti-oxidants, lubricants, binders, stabilisers, solubilisers, surfactants, masking agents, colouring agents, flavouring agents or sweetening agents can be found in standard pharmaceutical texts, for example, Remington's 'The Science and Practice of Pharmacy' (Ed. A. Adejare), 23rd Edition (2020), Academic Press.

A pharmaceutically acceptable excipient may be a compound or a combination of compounds entering into a pharmaceutical composition which does not provoke secondary reactions and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

The pharmaceutical compositions/medicaments according to the present disclosure may be formulated for administration to a subject, *e.g*. administration via a route of administration as appropriate for the nature of the therapeutic agent and the disease to be treated/prevented. In some embodiments, a pharmaceutical composition/medicament may be formulated for parenteral, systemic, intravascular, intravenous, intra-arterial, intramuscular, topical, intracavitary, intrathecal, intraocular, intraconjunctival, intratumoral, subcutaneous, intradermal, oral or transdermal administration. In some embodiments, a pharmaceutical composition/medicament may be formulated for administration by injection or infusion, or administration by ingestion.

For intra-venous administration, *e.g*. by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (*e.g*. Zn-protein complexes); and/or non-ionic surfactants, such as TWEENTM, PLURONICSTM or polyethylene glycol (PEG).

### Sequences

| **SEQ ID NO** | **DESRIPTION** | **SEQUENCE** |
|---|---|---|
| 1 | Hu36 IgG1-HC with signal sequence | METDTLLLWVLLLWVPGSTGQVQLVQSGAEVKKPGASVKVSCKASGYTF TENIIHWVRQAPGQGLEWMGWFHPGSGSIKYNEKFKDRVTMTADTSTST VYMELSSLRSEDTAVYYCARHGGTGRGAMDYWGQGTLVTVSSASTKGP SVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKT HTCPPCPAPPVAGPSVFLFPPKPKDTLMISRTPEVTCVVVDVSHEDPEVK FNWYVDGVEVHNAKTKPREEQYNSTYRVVSVLTVLHQDWLNGKEYKCK VSNKGLPSSIEKTISKAKGQPREPQVYTLPPSRDELTKNQVSLTCLVKGFY PSDIAVEWESNGQPENNYKTTPPVLDSDGSFFLYSKLTVDKSRWQQGNV FSCSVMHEALHNHYTQKSLSLSPGK |
| 2 | Hu36 IgG1-LC with signal sequence | METDTLLLWVLLLWVPGSTGDIQMTQSPSSLSASVGDRVTITCRASKSVS TSAYSYMHWYQQKPGKAPKLLIYLASNLESGVPSRFSGSGSGTDFTLTIS SLQPEDFATYYCQHSRELPYTFGQGTKLEIKRTVAAPSVFIFPPSDEQLKS GTASVVCLLNNFYPREAKVQWKVDNALQSGNSQESVTEQDSKDSTYSLS STLTLSKADYEKHKVYACEVTHQGLSSPVTKSFNRGEC |
| 3 | Hu36 IgG1-HC | QVQLVQSGAEVKKPGASVKVSCKASGYTFTENIIHWVRQAPGQGLEWM GWFHPGSGSIKYNEKFKDRVTMTADTSTSTVYMELSSLRSEDTAVYYCA RHGGTGRGAMDYWGQGTLVTVSSASTKGPSVFPLAPSSKSTSGGTAAL GCLVKDYFPEPVTVSWNSGALTSGVHTFPAVLQSSGLYSLSSVVTVPSSS LGTQTYICNVNHKPSNTKVDKKVEPKSCDKTHTCPPCPAPPVAGPSVFLF PPKPKDTLMISRTPEVTCVVVDVSHEDPEVKFNWYVDGVEVHNAKTKPR EEQYNSTYRVVSVLTVLHQDWLNGKEYKCKVSNKGLPSSIEKTISKAKGQ PREPQVYTLPPSRDELTKNQVSLTCLVKGFYPSDIAVEWESNGQPENNY KTTPPVLDSDGSFFLYSKLTVDKSRWQQGNVFSCSVMHEALHNHYTQKS LSLSPGK |
| 4 | Hu36 IgG1-LC | DIQMTQSPSSLSASVGDRVTITCRASKSVSTSAYSYMHWYQQKPGKAPK LLIYLASNLESGVPSRFSGSGSGTDFTLTISSLQPEDFATYYCQHSRELPY TFGQGTKLEIKRTVAAPSVFIFPPSDEQLKSGTASVVCLLNNFYPREAKVQ WKVDNALQSGNSQESVTEQDSKDSTYSLSSTLTLSKADYEKHKVYACEV THQGLSSPVTKSFNRGEC |
| 5 | Hu36 IgG1 VH | QVQLVQSGAEVKKPGASVKVSCKASGYTFTENIIHWVRQAPGQGLEWM GWFHPGSGSIKYNEKFKDRVTMTADTSTSTVYMELSSLRSEDTAVYYCA RHGGTGRGAMDYWGQGTLVTVSS |
| 6 | Hu36 IgG1 VL | DIQMTQSPSSLSASVGDRVTITCRASKSVSTSAYSYMHWYQQKPGKAPK LLIYLASNLESGVPSRFSGSGSGTDFTLTISSLQPEDFATYYCQHSRELPY TFGQGTKLEIKR |
| 7 | Hu36 IgG1 HC-CDR1 | ENIIH |
| 8 | Hu36 IgG1 HC-CDR2 | WFHPGSGSIKYNEKFKD |
| 9 | Hu36 IgG1 HC-CDR3 | HGGTGRGAMDY |
| 10 | Hu36 IgG1 LC-CDR1 | RASKSVSTSAYSYMH |
| 11 | Hu36 IgG1 LC-CDR2 | LASNLES |
| 12 | Hu36 IgG1 LC-CDR3 | QHSRELPYT |
| 13 | Human FAP (Uniprot #Q12884) | MKTWVKIVFGVATSAVLALLVMCIVLRPSRVHNSEENTMRALTLKDILNGT FSYKTFFPNWISGQEYLHQSADNNIVLYNIETGQSYTILSNRTMKSVNASN YGLSPDRQFVYLESDYSKLWRYSYTATYYIYDLSNGEFVRGNELPRPIQY LCWSPVGSKLAYVYQNNIYLKQRPGDPPFQITFNGRENKIFNGIPDWVYE EEMLATKYALWWSPNGKFLAYAEFNDTDIPVIAYSYYGDEQYPRTINIPYP KAGAKNPVVRIFIIDTTYPAYVGPQEVPVPAMIASSDYYFSWLTWVTDERV CLQWLKRVQNVSVLSICDFREDWQTWDCPKTQEHIEESRTGWAGGFFV STPVFSYDAISYYKIFSDKDGYKHIHYIKDTVENAIQITSGKWEAINIFRVTQ DSLFYSSNEFEEYPGRRNIYRISIGSYPPSKKCVTCHLRKERCQYYTASFS DYAKYYALVCYGPGIPISTLHDGRTDQEIKILEENKELENALKNIQLPKEEIK KLEVDEITLWYKMILPPQFDRSKKYPLLIQVYGGPCSQSVRSVFAVNWISY LASKEGMVIALVDGRGTAFQGDKLLYAVYRKLGVYEVEDQITAVRKFIEM GFIDEKRIAIWGWSYGGYVSSLALASGTGLFKCGIAVAPVSSWEYYASVY TERFMGLPTKDDNLEHYKNSTVMARAEYFRNVDYLLIHGTADDNVHFQN SAQIAKALVNAQVDFQAMWYSDQNHGLSGLSTNHLYTHMTHFLKQCFSL SD |
| 14 | Murine FAP (Uniprot #P97321) | MKTWLKTVFGVTTLAALALVVICIVLRPSRVYKPEGNTKRALTLKDILNGTF SYKTYFPNWISEQEYLHQSEDDNIVFYNIETRESYIILSNSTMKSVNATDYG LSPDRQFVYLESDYSKLWRYSYTATYYIYDLQNGEFVRGYELPRPIQYLC WSPVGSKLAYVYQNNIYLKQRPGDPPFQITYTGRENRIFNGIPDWVYEEE MLATKYALWWSPDGKFLAYVEFNDSDIPIIAYSYYGDGQYPRTINIPYPKA GAKNPVVRVFIVDTTYPHHVGPMEVPVPEMIASSDYYFSWLTWVSSERV CLQWLKRVQNVSVLSICDFREDWHAWECPKNQEHVEESRTGWAGGFFV |
| | | STPAFSQDATSYYKIFSDKDGYKHIHYIKDTVENAIQITSGKWEAIYIFRVTQ DSLFYSSNEFEGYPGRRNIYRISIGNSPPSKKCVTCHLRKERCQYYTASFS YKAKYYALVCYGPGLPISTLHDGRTDQEIQVLEENKELENSLRNIQLPKVEI KKLKDGGLTFWYKMILPPQFDRSKKYPLLIQVYGGPCSQSVKSVFAVNWI TYLASKEGIVIALVDGRGTAFQGDKFLHAVYRKLGVYEVEDQLTAVRKFIE MGFIDEERIAIWGWSYGGYVSSLALASGTGLFKCGIAVAPVSSWEYYASIY SERFMGLPTKDDNLEHYKNSTVMARAEYFRNVDYLLIHGTADDNVHFQN SAQIAKALVNAQVDFQAMWYSDQNHGISSGRSQNHLYTHMTHFLKQCFS LSD |
| 15 | Human FAP extracellular region (Uniprot #Q12884, positions 26 to 760) | LRPSRVHNSEENTMRALTLKDILNGTFSYKTFFPNWISGQEYLHQSADNNI VLYNIETGQSYTILSNRTMKSVNASNYGLSPDRQFVYLESDYSKLWRYSY TATYYIYDLSNGEFVRGNELPRPIQYLCWSPVGSKLAYVYQNNIYLKQRP GDPPFQITFNGRENKIFNGIPDWVYEEEMLATKYALWWSPNGKFLAYAEF NDTDIPVIAYSYYGDEQYPRTINIPYPKAGAKNPVVRIFIIDTTYPAYVGPQE VPVPAMIASSDYYFSWLTWVTDERVCLQWLKRVQNVSVLSICDFREDWQ TWDCPKTQEHIEESRTGWAGGFFVSTPVFSYDAISYYKIFSDKDGYKHIH YIKDTVENAIQITSGKWEAINIFRVTQDSLFYSSNEFEEYPGRRNIYRISIGS YPPSKKCVTCHLRKERCQYYTASFSDYAKYYALVCYGPGIPISTLHDGRT DQEIKILEENKELENALKNIQLPKEEIKKLEVDEITLWYKMILPPQFDRSKKY PLLIQVYGGPCSQSVRSVFAVNWISYLASKEGMVIALVDGRGTAFQGDKL LYAVYRKLGVYEVEDQITAVRKFIEMGFIDEKRIAIWGWSYGGYVSSLALA SGTGLFKCGIAVAPVSSWEYYASVYTERFMGLPTKDDNLEHYKNSTVMA RAEYFRNVDYLLIHGTADDNVHFQNSAQIAKALVNAQVDFQAMWYSDQN HGLSGLSTNHLYTHMTHFLKQCFSLSD |
| 16 | Murine FAP extracellular region (Uniprot #P97321 positions 26 to 761) | LRPSRVYKPEGNTKRALTLKDILNGTFSYKTYFPNWISEQEYLHQSEDDNI VFYNIETRESYIILSNSTMKSVNATDYGLSPDRQFVYLESDYSKLWRYSYT ATYYIYDLQNGEFVRGYELPRPIQYLCWSPVGSKLAYVYQNNIYLKQRPG DPPFQITYTGRENRIFNGIPDWVYEEEMLATKYALWWSPDGKFLAYVEFN DSDIPIIAYSYYGDGQYPRTINIPYPKAGAKNPVVRVFIVDTTYPHHVGPME VPVPEMIASSDYYFSWLTWVSSERVCLQWLKRVQNVSVLSICDFREDWH AWECPKNQEHVEESRTGWAGGFFVSTPAFSQDATSYYKIFSDKDGYKHI HYIKDTVENAIQITSGKWEAIYIFRVTQDSLFYSSNEFEGYPGRRNIYRISIG NSPPSKKCVTCHLRKERCQYYTASFSYKAKYYALVCYGPGLPISTLHDGR TDQEIQVLEENKELENSLRNIQLPKVEIKKLKDGGLTFWYKMILPPQFDRS KKYPLLIQVYGGPCSQSVKSVFAVNWITYLASKEGIVIALVDGRGTAFQGD KFLHAVYRKLGVYEVEDQLTAVRKFIEMGFIDEERIAIWGWSYGGYVSSLA LASGTGLFKCGIAVAPVSSWEYYASIYSERFMGLPTKDDNLEHYKNSTVM ARAEYFRNVDYLLIHGTADDNVHFQNSAQIAKALVNAQVDFQAMWYSDQ NHGISSGRSQNHLYTHMTHFLKQCFSLSD |
| 17 | Signal sequence | METDTLLLWVLLLWVPGSTG |

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1: Comparative in vitro activity of deruxtecan versus cytolysin payload

The cytotoxic activity of A1B1 cytolysin (payload), TAM558 (A1B1 payload-linker), Exatecan (payload) and Deruxtecan (Exatecan payload-linker) was analyzed via cell-viability assay using the FAP-transfected human fibrosarcoma cell line HT1080-FAP.

All compounds showed a concentration-dependent killing of the HT1080-FAP cells. From the two payloads tested, A1B1 cytolysin, from the tubulysin family, showed the best activity with an IC50 value of 0.06 nM, followed by Exatecan with a value of 0.4 nM, approximately ten times less active than A1B1.

Similarly, TAM558 showed the best activity from both payload-linkers, with an IC50 value of 710.5 nM compared to 3343 nM for Deruxtecan, almost 5 times less active.

**Table 1: Bioactivity of Deruxtecan, Exatecan, TAM558 and A1B1 on HT1080-FAP cell line.**

| **Payloads** | **IC₅₀ HT1080-FAP (nM)** |
|---|---|
| TAM558 | 710.5 |
| A1B1 | 0.06 |
| Deruxtecan | 3343 |
| Exatecan | 0.4 |

### EXAMPLE 2: Generation and production of anti-FAP Deruxtecan ADC

Generation and production of humanized anti-FAP antibody was carried out as described in Example 1 of WO 2015/118030 A2.

For the obtention of anti-FAP-Deruxtecan ADC (antibody-drug conjugate) (OMTX805), the anti-FAP IgG antibody described previously (WO 2015/118030 A2), was conjugated with the linker-drug GGFG-DXd, through a non-site specific and thiol-based reaction on the cysteine residues of the antibody.

The antibody was reduced by 9 TCEP/mAb equivalents for 16 hours and conjugated with 13 Drug/mAb equivalents for 1 hour. The conjugation was followed by Ultrafiltration and formulation in 20 mM Histidine/Histidine-HCl, 8% sucrose, pH 6.0. The final protein was concentrated to 13.14 mg/mL in formulation buffer.

The final anti-FAP-Deruxtecan ADC product (OMTX805) obtained presented a drug-antibody ratio (DAR) of 8 analyzed by HIC-HPLC. The purity was 98.99% of main peak and 1.01% of HMW. Free drug analyzed by HIC-HPLC was <0.05% and endotoxin content was <0.091 EU/mL.

**Table 2: Main characteristics of OMTX805 and OMTX705 Antibody-Drug Conjugates**

| **ADC** | **Conc. (mg/mL)** | **HIC-DAR** | **Monomer %** | **HMW%** | **Free drug (mol/mol %)** | **Endo (EU/mg)** |
|---|---|---|---|---|---|---|
| Anti-FAP-cytolisin (OMTX705) | 27.1 | 3.8 | 97 | 0.6 | nd. | 0.96 |
| Anti-FAP-Deruxtecan (OMTX805) | 13.14 | 8.00 | 98.99 | 1.01 | <0.05 | <0.091 |

| | | | | | | |
|---|---|---|---|---|---|---|
| nd =not detected | | | | | | |

### EXAMPLE 3: In vitro binding and activity of anti-FAP deruxtecan ADC

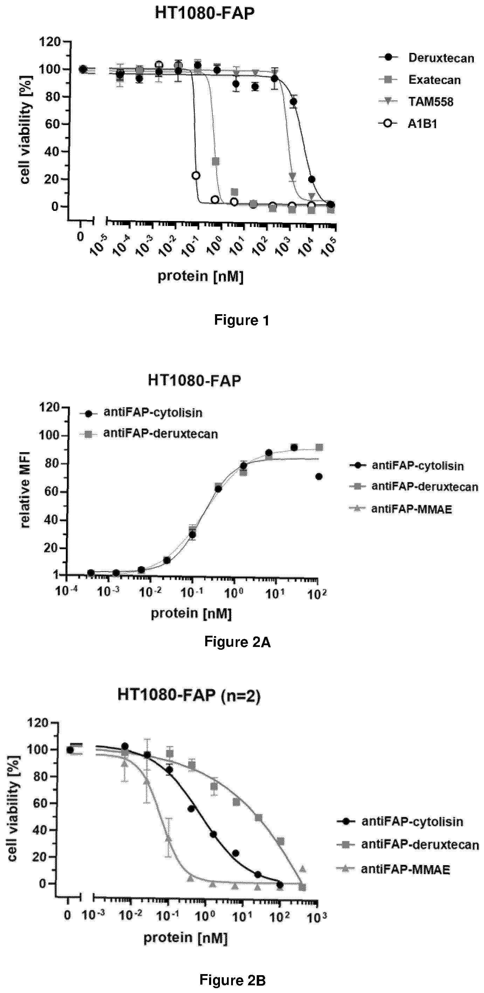

FAP-specific cell binding of anti-FAP-Deruxtecan (OMTX805) compared to anti-FAP-cytolisin (OMTX705) was analyzed via flow cytometry using FAP-expressing HT1080-FAP cells. Bound antibodies were detected using PE-labelled anti-human Fc secondary antibody and median fluorescence intensity (MFI) was measured. Anti-FAP-cytolisin (OMTX705) and anti-FAP-deruxtecan (OMTX805) showed a similar concentration-dependent binding with EC50 values of 0.17 nM for OMTX705 and 0.18 nM for OMTX805. (Figure 2a).

Both OMTX805 and OMTX705 ADCs together with an anti-FAP-MMAE (OMTX105) ADC were all tested in a cell-based cytotoxicity assay using HT1080-FAP and HT1080-WT cells. On HT1080-FAP cells, all ADCs showed a concentration-dependent killing of these cells after 3-day incubation with IC₅₀ values of 64 pM for OMTX105, 750 pM for OMTX705 and 80.685 pM for OMTX805 on HT1080-FAP cells. In HT1080-WT cells, a reduced activity was observed for all 3 ADCs with IC50 values of 77.150 pM for OMTX105, 61.795 pM for OMTX705 and 113.300 pM for OMTX805 (Figure 2b) (Table 3).

In agreement with the lowest activity of the payload (Figure 1), and although all 3 anti-FAP ADCs showed similar FAP binding, OMTX805, even with a DAR value of 8, presented the lowest *in vitro* activity and specificity ratio, compared to cytolysin payload or cytolysin and MMAE anti-FAP conjugates.

**Table 3: in vitro binding and cytotoxic activity values of anti-FAP ADCs**

| ADC | Cell binding EC50 (pM) | IC50 (pM) | | Specificity ratio |
|---|---|---|---|---|
| | HT1080FAP | HT1080FAP | HT1080WT | |
| OMTX105 | n.d. | 64 ± 33 | 77.150 ± 311 | 1.205 |
| OMTX705 | 170 | 750 ± 93 | 61.795 ± 4,575 | 82 |
| OMTX805 | 180 | 80.685 ± 68.469 | 113.300 ± 6,081 | 1.4 |

### EXAMPLE 4: Comparative in vivo antitumoral efficacy of Deruxtecan versus cytolysin anti-FAP antibody drug conjugates

The antitumor activity of OMTX805 (anti-FAP-Deruxtecan) vs OMTX705 (anti-FAP-cytolysin) was evaluated in the CO-04-0727 colorectal carcinoma patient derived xenograft model in 6-8 weeks old female BALB/c nude mice.

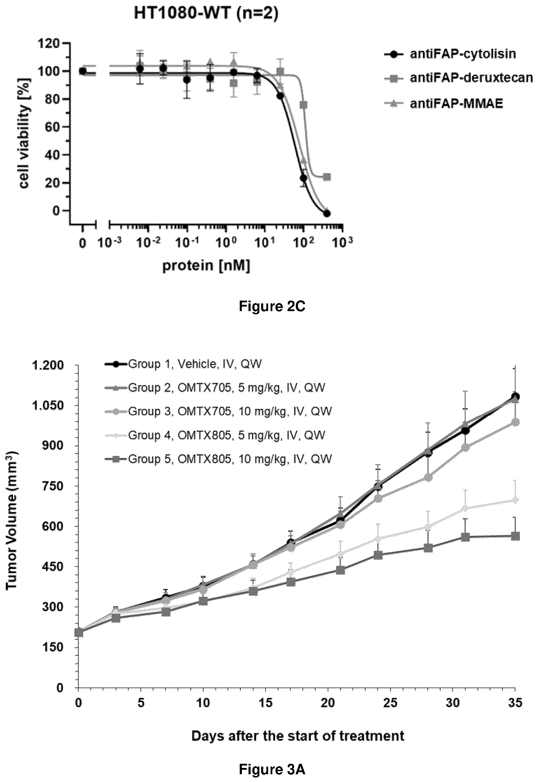

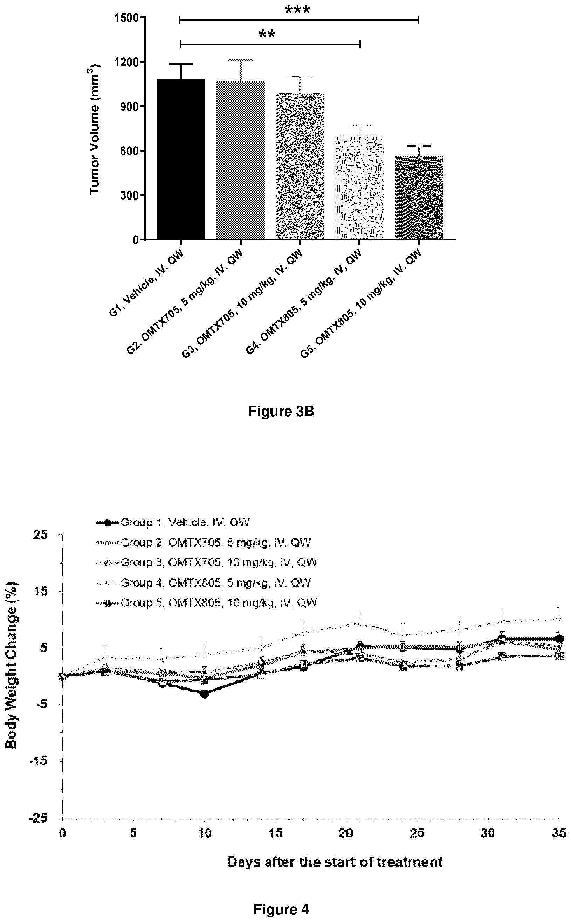

OMTX805 and OMTX705 were administered intravenously at 5 and 10 mg/kg both once a week for 4 weeks. Both OMTX805 and OMTX705 demonstrated anti-tumor efficacy. OMTX705 anti-FAP-cytolysin ADC showed a percentage tumor growth inhibition of 10.85% at 10 mg/kg versus control group (Figure 3A and B). Unexpectedly with respect of previous *in vitro* data (Figures 1 & 2), OMTX805 demonstrated higher anti-tumor efficacy than OMTX705. OMTX805 at 5 and 10 mg/kg showed 43.89% and 59.05% of tumor growth inhibition, respectively, versus control group.

Both conjugates were well tolerated at the doses tested (5 and 10 mg/kg), showing comparable weight loss (OMTX805 10 mg/kg, OMTX705 5 mg/kg and OMTX705 10 mg/kg), or decreased weight loss (OMTX805 5 mg/kg) to the control condition over the 35-day test period (Figure 4). OMTX805 at a dosage of 5 mg/kg demonstrated the highest tolerance in mice out of all test conditions, with no weight loss detected after 35 days. This highlights the high tolerance of OMTX805 in mice.

These findings support for the first time the highest efficacy of Deruxtecan payloads compared with microtubule inhibitor payloads when conjugated with anti-FAP antibodies in indications of colorectal carcinomas.

### References

A number of publications are cited above in order to more fully describe and disclose the invention and the state of the art to which the invention pertains. Full citations for these references are provided below. The entirety of each of these references is incorporated herein.
1. Dumontet, C., Reichert, J. M., Senter, P. D., Lambert, J. M. & Beck, A. Antibody-drug conjugates come of age in oncology. Nature Reviews Drug Discovery vol. 22 Preprint at
   https://doi.org/10.1038/s41573-023-00709-2 (2023).
2. Hafeez, U., Parakh, S., Gan, H. K. & Scott, A. M. Antibody⇓drug conjugates for cancer therapy. Molecules vol. 25 Preprint at https://doi.org/10.3390/molecules25204764 (2020).
3. Liu, K. et al. A review of the clinical efficacy of FDA-approved antibody-drug conjugates in human cancers. Molecular Cancer vol. 23 Preprint at https://doi.org/10.1186/s12943-024-01963-7 (2024).
4. Maecker, H., Jonnalagadda, V., Bhakta, S., Jammalamadaka, V. & Junutula, J. R. Exploration of the antibody-drug conjugate clinical landscape. mAbs vol. 15 Preprint at
   https://doi.org/10.1080/19420862.2023.2229101 (2023).
5. Fu, Z., Li, S., Han, S., Shi, C. & Zhang, Y. Antibody drug conjugate: the "biological missile" for targeted cancer therapy. Signal Transduction and Targeted Therapy vol. 7 Preprint at
   https://doi.org/10.1038/s41392-022-00947-7 (2022).
6. Wang, Z., Li, H., Gou, L., Li, W. & Wang, Y. Antibody-drug conjugates: Recent advances in payloads. Acta Pharmaceutica Sinica B vol. 13 Preprint at https://doi.org/10.1016/j.apsb.2023.06.015 (2023).
7. Čermák, V. et al. Microtubule-targeting agents and their impact on cancer treatment. *European Journal of Cell Biology* vol. 99 Preprint at https://doi.org/10.1016/j.ejcb.2020.151075 (2020).
8. Chen, H., Lin, Z., Arnst, K. E., Miller, D. D. & Li, W. Tubulin inhibitor-based antibody-drug conjugates for cancer therapy. Molecules vol. 22 Preprint at https://doi.org/10.3390/molecules22081281 (2017).
9. Doronina, S. O. & Senter, P. D. CHAPTER 4: Auristatin Payloads for Antibody-Drug Conjugates (ADCs). in RSC Drug Discovery Series vols 2019-January (2019).
10. Xiangrong, X., Yao, L. & Yao, A. The Recent Developments of ADCs with the Tubulysins as the Payloads. Mini-Reviews in Medicinal Chemistry 23, (2023).
11. Swanton, C., Tomlinson, !. & Downward, J. Chromosomal instability, colorectal cancer and taxane resistance. Cell Cycle vol. 5 Preprint at https://doi.org/10.4161/cc.5.8.2682 (2006).
12. Conilh, L., Sadilkova, L., Viricel, W. & Dumontet, C. Payload diversification: a key step in the development of antibody-drug conjugates. Journal of Hematology and Oncology vol. 16 Preprint at https://doi.org/10.1186/s13045-022-01397-y (2023).
13. Douillard, J. Y. et al. Irinotecan combined with fluorouracil compared with fluorouracil alone. as first-line treatment for metastatic colorectal cancer: a multicentre randomised trial. Lancet355, (2000).
14. Saltz, L. B. et al. Irinotecan plus fluorouracil and leucovorin for metastatic colorectal cancer. Irinotecan Study Group. N Engl J Med 343, (2000).
15. Senior, M. Cancer-targeting antibody-drug conjugates drive dealmaking frenzy. Nature Biotechnology vol. 42 362-366 Preprint at https://doi.org/10.1038/s41587-024-02168-5 (2024).
16. Han, S. et al. The Potential of Topoisomerase Inhibitor-Based Antibody-Drug Conjugates. Pharmaceutics vol. 14 Preprint at https://doi.org/10.3390/pharmaceutics14081707 (2022).
17. Meric-Bernstam, F. et al. Efficacy and Safety of Trastuzumab Deruxtecan in Patients With HER2-Expressing Solid Tumors: Primary Results From the DESTINY-PanTumor02 Phase II Trial. Journal of Clinical Oncology 42, 47-58 (2024).
18. Tsuchikama, K., Anami, Y., Ha, S. Y. Y. & Yamazaki, C. M. Exploring the next generation of antibody-drug conjugates. Nature Reviews Clinical Oncology vol. 21 203-223 Preprint at https://doi.org/10.1038/s41571-023-00850-2 (2024).
19. Khoury, R. et al. Mechanisms of Resistance to Antibody-Drug Conjugates. International Journal of Molecular Sciences vol. 24 Preprint at https://doi.org/10.3390/ijms24119674 (2023).
20. Xiao, Y. & Yu, D. Tumor microenvironment as a therapeutic target in cancer. Pharmacology and Therapeutics vol. 221 Preprint at https://doi.org/10.1016/j.pharmthera.2020.107753 (2021).
21. Valkenburg, K. C., De Groot, A. E. & Pienta, K. J. Targeting the tumour stroma to improve cancer therapy. Nature Reviews Clinical Oncology vol. 15 Preprint at https://doi.org/10.1038/s41571-018-0007-1 (2018).
22. Xu, M., Zhang, T., Xia, R., Wei, Y. & Wei, X. Targeting the tumor stroma for cancer therapy. Molecular Cancer vol. 21 Preprint at https://doi.org/10.1186/s12943-022-01670-1 (2022).
23. Xin, L. et al. Fibroblast Activation Protein-a as a Target in the Bench-to-Bedside Diagnosis and Treatment of Tumors: A Narrative Review. Frontiers in Oncology vol. 11 Preprint at https://doi.org/10.3389/fonc.2021.648187 (2021).
24. Zana, A. et al. A Comparative Analysis of Fibroblast Activation Protein-Targeted Small Molecule-Drug, Antibody-Drug, and Peptide-Drug Conjugates. Bioconjug Chem 34, (2023).
25. Fabre, M. et al. OMTX705, a novel FAP-targeting ADC demonstrates activity in chemotherapy and pembrolizumab-resistant solid tumor models. Clinical Cancer Research 26, (2020).
26. Zhang, Z. et al. From basic research to clinical application: targeting fibroblast activation protein for cancer diagnosis and treatment. Cellular Oncology Preprint at https://doi.org/10.1007/s13402-023-00872-z (2023).
For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. A conjugate having the formula I:
A-(L-D)ₚ (I)
or a pharmaceutically acceptable salt or solvent thereof,
wherein:
A is an antibody comprising
(i) a heavy chain variable (VH) region comprising the following CDRs:
HC-CDR1 having the amino acid sequence of SEQ ID NO:7
HC-CDR2 having the amino acid sequence of SEQ ID NO:8
HC-CDR3 having the amino acid sequence of SEQ ID NO:9
or a variant thereof having one amino acid variation in one or more of HC-CDR1, HC-CDR2, or HC-CDR3; and
(ii) a light chain variable (VL) region comprising the following CDRs:
LC-CDR1 having the amino acid sequence of SEQ ID NO:10
LC-CDR2 having the amino acid sequence of SEQ ID NO:11
LC-CDR3 having the amino acid sequence of SEQ ID NO:12
or a variant thereof having one amino acid variation in one or more of LC-CDR1, LC-CDR2, or LC-CDR3;
L is linker
D is a drug comprising a topoisomerase inhibitor
p is 1 to 15.

2. The conjugate of claim 1, wherein A comprises:
(i) a heavy chain variable (VH) region comprising the following CDRs:
HC-CDR1 having the amino acid sequence of SEQ ID NO:7
HC-CDR2 having the amino acid sequence of SEQ ID NO:8
HC-CDR3 having the amino acid sequence of SEQ ID NO:9; and
(ii) a light chain variable (VL) region comprising the following CDRs:
LC-CDR1 having the amino acid sequence of SEQ ID NO:10
LC-CDR2 having the amino acid sequence of SEQ ID NO:11
LC-CDR3 having the amino acid sequence of SEQ ID NO:12.

3. The conjugate of claim 1 or claim 2, wherein A comprises a heavy chain variable (VH) region comprising an amino acid sequence having at least 90%, 95%, 99% or 100% sequence identity with SEQ ID NO:5, and/or wherein A comprises a light chain variable (VL) region comprising an amino acid sequence having at least 90%, 95%, 99% or 100% sequence identity with SEQ ID NO:6.

4. The conjugate of any one of claims 1 to 3, wherein A comprises a heavy chain comprising an amino acid sequence having at least 90%, 95%, 99% or 100% sequence identity with SEQ ID NO:3, and/or, wherein A comprises a light chain comprising an amino acid sequence having at least 90%, 95%, 99% or 100% sequence identity with SEQ ID NO:4.

5. The conjugate of any one of claims 1 to 4, wherein D is of formula II: 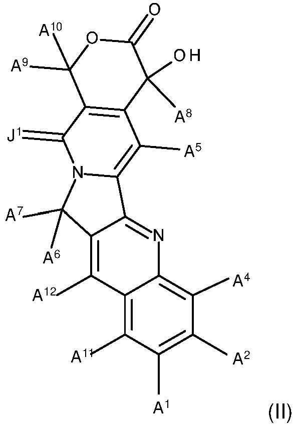
A¹ and A² are each independently selected from the group consisting of halo, hydrogen, C₁-C₃ alkyl, phenyl, hydroxy, C₁-C₃ alkoxy,
or wherein A¹ and A² together with the atoms to which they are bonded form a 5-6 membered fused ring;
either
A¹¹ is H;
A¹² is -(C₁₋₆ alkyl)-A³ or - A³; or
A¹¹ and A¹² together with the carbon atoms to which they are attached form a ring substituted with A³ at any position on the ring^{,} preferably the ring structure is 4, 5, 6, 7 or 8 atoms in size, more preferably 5, 6, 7 or 8 atoms in size; preferably the ring is a carbocyclic ring;
A³ is 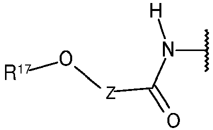
wherein
Z is a bond or a C₁-C₆ alkyl chain or a substituted or unsubstituted aryl group or wherein Z is X₁-Ar, wherein the Ar group is directly attached to the OR¹⁷ group.
wherein X₁ is a C₁-C₆ alkyl chain and Ar is a substituted or unsubstituted aryl group;
A⁴, A⁵, A⁶ A⁷, A⁹ and A¹⁰ are each individually hydrogen or a C₁-C₃ alkyl;
A⁸ is hydrogen or a C₁-C₄ alkyl;
R¹⁷ is a bond, -CH₂X', -CH₂NHX', -CH₂NHC(O)X' -C(O)NHNHC(O)X', -C(O)X', -C(O)NHNHX', -OX', -NHX' or -SX', wherein X' is a bond to linker L
and
J¹ is O, S, or Se.

6. The conjugate of any one of claims 1 to 5, wherein D is Exatecan.

7. The conjugate of any one of claims 1 to 6, wherein L comprises a GGFG linker, optionally wherein L comprises an aminomethoxy group.

8. The conjugate of any one of claims 1 to 7, wherein L-D has the following structure: 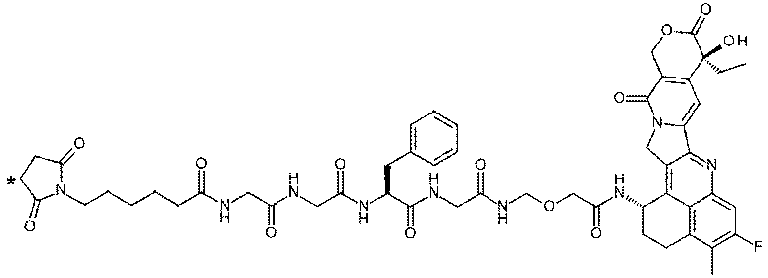 wherein * indicates the site of attachment to A.

9. The conjugate of any one of claims 1 to 8, wherein p is 5, 6, 7, 8 or 9.

10. The conjugate of any one of claims 1 to 9, for use in medicine.

11. The conjugate of any one of claims 1 to 10, for use in a method of treatment of
(i) cancer in a mammalian subject, optionally wherein the cancer is colorectal cancer, sarcoma, fibrosarcoma, leiomyosarcoma, oesophageal cancer, breast cancer, melanoma, gastric cancer, head and neck cancer, ovarian cancer, bladder cancer, lung cancer, pancreatic cancer, cholangiocarcinoma, or mesothelioma; or
(ii) an inflammatory or fibrotic condition in a mammalian subject, optionally wherein the inflammatory or fibrotic condition is liver fibrosis, lung fibrosis, kidney fibrosis, cardiac fibrosis, Crohn's disease, or arthritis.

12. The conjugate for use according to claim 11, wherein the cancer is a colorectal cancer having FAP-expressing tumor cells and/or FAP-expressing stromal cells, optionally wherein the colorectal cancer is microsatellite-stable (MSS) colorectal cancer.

13. The conjugate for use according to claim 11 or claim 12, wherein the cancer is a metastatic cancer and wherein the primary cancer of the metastatic cancer comprises colorectal cancer or MSS colorectal cancer, optionally wherein the metastatic cancer comprises peritoneal metastases or liver metastases.

14. The conjugate for use according to claim 12 or claim 13, wherein the method comprises simultaneous, sequential or separate administration of one or more other anti-cancer agents; optionally wherein the one or more anti-cancer agents comprise a cytotoxic chemotherapy agent, an anti-angiogenic agent or an immunotherapeutic agent.

15. The conjugate for use according to claim 14, wherein the one or more other anti-cancer agents comprise anti-programmed cell death protein 1 (PD-1) antibodies, or anti-programmed death-ligand 1 (PD-L1) antibodies, anti-CTLA-4 antibodies, or multi-kinase inhibitors, optionally wherein the one or more other anti-cancer agents comprise Nivolumab (MDX1106) or Pembrolizumab (MK-3475), Tislelizumab, Regorafenib or Pamufetinib (TAS-115).
